# EUROPEAN PATENT APPLICATION

(11) **EP 1 749 839 A1**
(43) Date of publication of application: **07.02.2007**
(21) Application number: 05106770.0
(22) Date of filing: 22.07.2005
(51) Int. Cl.: C07K 16/12, C12N 15/82, A61K 39/40

(54) **Antigen binding polypeptides against F4(K88) fimbriae**

(71) Applicant: Novoplant GmbH, 06466 Gatersleben (DE)
(72) Inventor: Zimmermann, Jana, Dr., 06766 Bobbau (DE); Giersberg, Martin, Dr., 06484 Quedlinburg (DE); Riehl, Marcus, Dr., 06502 Thale (DE)
(74) Representative: Neuefeind, Regina

(57) **Abstract**

The present invention relates to antigen-binding polypeptides, particularly antibody fragments, and especially single-chain antibodies, which specifically bind to bacterial F4 (K88)-fimbriae. The polypeptides may neutralize the attachment of F4 (K88) fimbriae to enterocytes and/or have a protective and/or curative effect on infection of mammals, especially pigs, with bacteria expressing F4 (K88)-fimbriae. Furthermore, the invention relates to polynucleotides encoding said F4 (K88)-binding polypeptides, vectors comprising these polynucleotides and prokaryotic or eukaryotic host cells comprising these polynucleotides or vectors. Also provided are transgenic plants or parts thereof and microorganisms expressing the polynucleotides of the present invention. The plants or plant parts expressing the polypeptides may be used as fodder for mammals, and especially pigs. Furthermore, the present invention provides compositions comprising said F4 (K88)-binding polypeptides, and methods of producing said polypeptides.

## Description

The present invention relates to antigen-binding polypeptides, particularly antibody fragments, and especially single-chain antibodies, which specifically bind to bacterial F4 (K88)-fimbriae. The polypeptides may neutralize the attachment of F4 (K88) fimbriae to enterocytes and/or have a protective and/or curative effect on infection of mammals, especially pigs, with bacteria expressing F4 (K88)-fimbriae. Furthermore, the invention relates to polynucleotides encoding said F4 (K88)-binding polypeptides, vectors comprising these polynucleotides and prokaryotic or eukaryotic host cells comprising these RN:GH polynucleotides or vectors. Also provided are transgenic plants or parts thereof and microorganisms expressing the polynucleotides of the present invention. The plants or plant parts expressing the polypeptides may be used as fodder for mammals, and especially pigs. Furthermore, the present invention provides compositions comprising said F4 (K88)-binding polypeptides, and methods of producing said polypeptides.

### BACKGROUND

The bacterial surface structures "F4 (K88) fimbriae" are the most important and frequent fimbrial antigens of pathogenic *E.coli* in piglets. The F4-fimbriae are composed of two different antigenic components, resulting in three antigen variants which are called K88ab, K88ac and K88ad (F4ab, F4ac and F4ad).

First step of the disease process of intestine associated *E.coli*-infections after oral uptake of the pathogen is the attachment of *E.coli* cells to the mucosal cells of the small intestine. The fimbriae attach to specific receptors of the enterocytes. The *E.coli* cells are thus prevented to be shed from the small intestine. After attachment bacteria multiply rapidly. The production of toxins and other pathogenic factors results in damage of the host mucosa. The pathology of the illnesses caused by *E.coli* varies and primarily depends on the different virulence factors, surface structures and toxins (Bosworth B.T. Infect. Immun. 1996 Jan;64(1):55-60).

Fimbriae can be discriminated by immune electrophoresis according to their serological activity. Different combinations of the fimbriae F4 (K88), F5 (K99), F6 (987P), F41 (Adhaesin) and F18 (F107, 2134P, 8813) and also of fimbriae with one or more toxins are possible (Mainil J.G. Vet. Microbiol. 1998 Aug 15;62(4):291-301). Thus frequently *E.coli* are isolated which carry F4-fimbriae and express the heat-stable and heat-labile enterotoxins, as well as *E.coli* expressing F5- or F6-fimbriae in combination with the heat-stable enterotoxins.

*E.coli* with F5/F6/F41 fimbriae are primarily detected in the distal jejunum and in the ileum (Bertschinger H.U. Vet. Microbiol. 1993 May;35(1-2):79-89), while *E.coli* with F4-fimbriae colonize the entire small intestine and the ileum (Jeyasingham M.D. Vet. Microbiol. 1999 Aug 31;68(3-4):219-34).

For some of the fimbriae a genetic resistance of the host was proven. Certain pig phenotypes exhibit a F4 (K88)-resistance which is recessively inherited, while the susceptibility for *E.coli* carrying F4-fimbriae is dominantly inherited (Gibbons et al., Theor. Appl. Genet. 1977, 51 :65-70; Rapacz et al., Anim. Genet. 1986, 17:305-321).

Piglets are very susceptible to F4 (K88)+ enterotoxigenic *E.coli*-induced neonatal and post-weaning diarrhoea. Further the bacteria tend to spread within a host population which leads to an important infectious load in the population. Thus, the ability to protect animals from *E.coli* infections and/or treat infected animals is of great importance.

Antibodies are proteins which are produced and secreted by B lymphocytes / plasma cells and which reversely and non-covalently bind to their specific antigen. They consist of four polypeptide chains, two identical heavy (H) chains of about 50-70 kD and two identical light (L) chains of about 25 kD. Each chain consists of a constant (C) and a variable (V) region. The constant region is composed of three to four constant domains in case of the heavy chain (CH₁ - CH₄) and one constant domain in case of the light chain (CL).

The variable region of the heavy chain (VH) and of the light chain (VL) are each composed of one domain, consisting of about 110 amino acids. The VH and the VL region form the antigen binding site. They each comprise three complementarity determining regions (CDR1, CDR2, CDR3) which represent the most variable regions of the antibody and which confer antigen binding specificity. The framework regions in between the CDRs are relatively conserved.

It has been shown that fragments of a whole antibody containing the CDR regions can still perform the binding function. These smaller molecules can be produced effectively e.g. in *E. coli,* yeasts and plant cells whereas whole IgG molecules still need to be produced in mammalian cell culture. Furthermore unwanted effects triggered by the constant part of the molecule are avoided, such as complement activation. However, the clearance rate is higher for antibody fragments, resulting in a short half-life upon systemic application.

An antibody "Fab" fragment (fragment antigen binding) is composed of a complete light chain linked to the N-terminal part of a heavy chain, consisting of the variable region (VH) and one constant domain (CH₁). The Fab fragment may be generated e.g. by papain hydrolysis of an antibody.

Gene technology methods such as phage display allow the production of a recombinant single-chain antibody "Fv" fragment (fragment variable) which is a truncated Fab fragment and consists of the variable region of a heavy chain and the variable region of a light chain, which are linked to each other by a synthetic peptide (scFv, single-chain fragment variable). "Phage display" is a standard technique for the presentation of distinct peptides, polypeptides or proteins on the surface of bacteriophages. It allows the identification of the exposed peptides, polypeptides or proteins via their binding properties. The technique is used for the establishment of libraries of peptide, polypeptide or protein variants such as different antibody fragments. For this, mammals such as mice are immunized against the antigen of interest and lymphocytes are isolated, e.g. from the spleen. Then, mRNA is isolated therefrom, and a cDNA library is generated by cloning the cDNA into a phage display vector.

The phage display library generated in this way contains recombinant scFv antibody fragments. The random library is screened for protein-protein interactions, e.g. for their antigen binding specificities. Interacting clones of the library are enriched, and the plasmid DNA from individual clones can be isolated and sequenced.

It is an object of the present invention to provide novel antigen-binding polypeptides, particularly antibody fragments, and especially single-chain antibodies, which specifically bind to bacterial F4 (K88)-fimbriae. These polypeptides preferably neutralize the attachment of F4 (K88) fimbriae to enterocytes and/or have a protective and/or curative effect on infection of mammals, especially pigs, with bacteria expressing F4 (K88)-fimbriae. A further object of the present invention is to provide polynucleotides and/or vectors encoding these polypeptides, prokaryotic or eukaryotic host cells comprising these polynucleotides and/or vectors, transgenic plants or parts thereof or microorganisms containing the polynucleotides and/or vectors of the present invention, as well as compositions comprising said F4 (K88)-binding polypeptides. Furthermore, the present invention also provides a method by which said F4 (K88)-binding polypeptides may be produced.

These and further objects are achieved by the subject-matter of the patent claims, which will become clear from the following description.

### DESCRIPTION

In a first aspect the present invention relates to an antigen-binding polypeptide which specifically binds to bacterial F4 (K88)-fimbriae. The term "antigen-binding polypeptide" as used herein means a polypeptide or protein which, according to the invention, specifically binds to bacterial F4 (K88). The term "specific binding" as used herein means that the polypeptide of the present invention does not show any significant binding to molecules other than bacterial F4 (K88) fimbriae or fragments thereof. Usually this binding is reversible and/or non-covalent.

Other preferred embodiments of the present invention are defined in the respective subclaims.

In one preferred embodiment, the antigen-binding polypeptide of the present invention binds to a polypeptide comprising the amino acid sequence of bacterial F4 (K88) ab, F4 (K88) ac and/or F4 (K88) ad (SEQ ID NOs: 1, 2 and/or 3) and/or fragments thereof.

In another aspect the present invention relates to an antigen-binding polypeptide comprising a first polypeptide comprising the antigen-binding portion of the variable region of an antibody heavy chain, and a second polypeptide comprising the antigen-binding portion of the variable region of an antibody light chain, wherein said antigen-binding polypeptide specifically binds to bacterial F4 (K88)-fimbriae.

The term "heavy chain" or "light chain" as used herein means the conventional antibody polypeptide heavy chain (H) or light chain (L), respectively. The term "variable region" as used herein means the conventional antibody variable region or variable domain or variable fragment (V or Fv) which confers antigen binding specificity. Variable regions employed in the invention may be derived from any germline or rearranged variable domain, or may be a synthetic variable domain based on consensus sequences of known variable domains. Usually the variable region of the heavy chain (VH) and the variable region of the light chain (VL) each comprise three complementarity determining regions CDR1, CDR2 and CDR3.

Seven antigen-binding polypeptides which specifically bind to bacterial F4 (K88) fimbriae have been generated via immunization of mice, cloned into a phage display library, tested for their F4 (K88)-binding specificity, isolated and sequenced. They carry the designations BA61, BA31, BA29, BA3, BA11, BA67 and BA5. These polypeptides are single-chain polypeptides. Their full length amino acid sequences are depicted in SEQ ID NOs: 4, 5, 6, 7, 8, 9 and 10, and their full length nucleotide sequences are depicted in SED ID NOs: 11, 12, 13, 14, 15, 16 and 17.

Surprisingly it was found that these polypeptides, all comprising an antigen-binding portion of a VH chain and an antigen-binding portion of a VL chain, are characterized by similar CDR1, CDR2 and CDR3 regions, especially in the VH CDR1 region (tables 1 and 2).
Therefore it was possible to characterize these F4 (K88)-binding single-chain polypeptides by means of amino acid and nucleotide consensus sequences of their CDRs. The locations of the CDRs were determined by reference to Kabat E.A. et al. (Sequences of Proteins of Immunological Interest, 4th Edition, U.S. Department of Health and Human Services, 1987) and updates thereof, now available on the Internet.

Table 1: Individual and consensus amino acid sequences of VH CDR1, VH CDR2 and VH CDR3 of the F4 (K88)-binding polypeptides BA61, BA31, BA29, BA3, BA11, BA67 and BAS. The most often occurring common amino acids are highlighted in bold letters. Amino acids of the consensus sequences in parentheses are optional. X₁= N or D; X₂ = A or V; X₃ = M or I; X₄ = S or H; X₅ = E, Y or V; X₆ = S or N; X₇ = T or P; X₈ = G, A or Y; X₉ = G, N or S; X₁₀ = S, D or G; X₁₁ = H, Y, N, F or S; X₁₂ = Y, K or N; X₁₃ = P or N; X₁₄ = D, E or Q; X₁₅ =T, K or S; X₁₆ = V or F; X₁₇ = T or K; X₁₈ = G or D; X₁₉ = T or V; X₂₀ = G, D or N; X₂₁= Y or E; X₂₂ = T, Y or N; X₂₃ = P, A or G; X₂₄ = F or M; X₂₅ = D or A; X₂₆ = Y or V; X₂₇=G or A; X₂₈ = H, Y or N; X₂₉ = Y or V; X₃₀ = N or D; X₃₁ = V or A; X₃₂ = I or M; X₃₃ = Y or V; X₃₄ = N or S; X₃₅ = P or T; X₃₆ = N or S; X₃₇ = D or G; X₃₈ = F or S; X₃₉ = K or N; X₄₀ = E or Q; X₄₁ = K or S; X₄₂ = G or D; X₄₃ = V or T; X₄₄ = G, D or N; X₄₅ = E or Y; X₄₆ = T, Y or N; X₄₇ = P, A or G; X₄₈ = F or M; X₄₉ = A or D; X₅₀ = S or K; X₅₁ = G or D; X₅₂ = D or N; X₅₃ = Y or N; X₅₄ = A or G; X₅₅ = A or V; X₅₆ = M or I; X₅₇ = S or H; X₅₈ = E or Y; X₅₉ = S or N; X₆₀ = T or P; X₆₁ = G, A or Y; X₆₂ = G or N; X₆₃ = S or D; X₆₄ = H, Y, N or F; X₆₅ = Y or K; X₆₆ = P or N; X₆₇ = D or E; X₆₈ = T or K; X₆₉ = V or F; X₇₀ = T or K; X₇₁ = T or V; X₇₂ = Y or E; X₇₃ = D or A; X₇₄ = Y or V.

| | VH CDR1 | SEQ ID No. | VH CDR2 | SEQ ID No. | VH CDR3 | SEQ ID No. |
|---|---|---|---|---|---|---|
| BA61 | **NYAMS** | 18 | **EIST**G**GS**H**TYYPDTVTG** | 24 | **TGY-----FDY** | 35 |
| BA31 | **NYAMS** | 18 | **EISTAGS**Y**TYYPDTVTG** | 25 | **TGY-----FDY** | 35 |
| BA29 | **NYAMS** | 18 | **EISTAGSNTYYPDTVTG** | 26 | **TGY-----FDV** | 36 |
| BA3 | **NYAMS** | 18 | **EISTAGSNTYYPDTVTG** | 26 | **TGY**-----**FD**V | 36 |
| BA11 | **NY**VIH | 19 | Y**I**NPYNDF**T**K**Y**NEKFK**G** | 27 | V**G**E---TP**F**A**Y** | 37 |
| BA67 | D**YAM**H | 20 | V**IST**YSGS**T**N**Y**NQSFK**G** | 28 | **T**D**Y**PYFYAM**DY** | 38 |
| BA5 | D**YAM**H | 20 | V**IST**YSGS**T**N**Y**NQKFKD | 29 | **T**N**Y**PYFNGM**DY** | 39 |

| cons.: | | | | | | |
|---|---|---|---|---|---|---|
| all | X₁**Y**X₂X₃X₄ | **21** | X₅**I**X₆X₇X₈X₉X₁₀X₁₁**T**X₁₂**Y** X₁₃X₁₄X₁₅X₁₆X₁₇X₁₈ | 30 | X₁₉X₂₀X₂₁ (PYF) (X₂₂X₂₃) X₂₄X₂₅ X₂₆ | **40** |
| BA61+31+ 29+3 | **NYAMS** | **18** | **EIST**X₂₇**GS**X₂₈**TYYPDTVTG** | **31** | **TGYFD**X₂₉ | **41** |
| BA11+67+ 5 | X₃₀**Y**X₃₁X₃₂ H | **22** | X₃₃**I**X₃₄X₃₅YX₃₆X₃₇X₃₈**T**X₃₉ **Y**NX₄₀X₄₁FKX₄₂ | 32 | X₄₃X₄₄X₄₅ (PYF) X₄₆X₄₇X₄₈X₄₉**Y** | **42** |
| BA67+5 | D**YAM**H | **20** | V**IST**YSGS**T**N**Y**NQX₅₀FKX₅₁ | 33 | **T**X₅₂**Y**PYFX₅₃X₅₄ M**DY** | **43** |
| BA61+31+ 29+3+11 | **NY**X₅₅X₅₆ X₅₇ | **23** | X₅₈**I**X₅₉X₆₀X₆₁X₆₂X₆₃X₆₄**T** X₆₅**Y**X₆₆X₆₇X₆₈X₆₉X₇₀**G** | 34 | X₇₁**G**X₇₂ (TP) **F** X₇₃X₇₄ | **44** |

The amino acid sequences which are designated in claims 5 to 10 refer to the SEQ ID NOs of table 1. For the purpose of clarification of the sequence protocol, the consensus sequences of table 1 which contain amino acids in parentheses (SEQ ID NOs: 40, 42 and 44) are defined in the sequence protocol as follows:
- SEQ ID NO: 40: X₁₉X₂₀X₂₁PYFX₂₂X₂₃X₂₄X₂₅X₂₆
- SEQ ID NO: 139: X₁₉X₂₀X₂₁X₂₄X₂₅X₂₆
- SEQ ID NO: 140: X₁₉X₂₀X₂₁X₂₂X₂₃X₂₄X₂₅X₂₆

- SEQ ID NO: 42: X₄₃X₄₄X₄₅PYFX₄₆X₄₇X₄₈X₄₉Y
- SEQ ID NO: 141: X₄₃X₄₄X₄₅X₄₆X₄₇X₄₈X₄₉Y

- SEQ ID NO: 44: X₇₁GX₇₂TPFX₇₃X₇₄
- SEQ ID NO: 142: X₇₁GX₇₂FX₇₃X₇₄

Table 2: Individual and consensus amino acid sequences of VL CDR1, VL CDR2 and VL CDR3 of the F4 (K88) binding polypeptides BA61, BA31, BA29, BA3, BA11, BA67 and BA5. The most often occurring common amino acids are highlighted in bold letters. Amino acids of the consensus sequences in parentheses are optional. X₁= K, R or S; X₂ = A or G; X₃ = Q or S; X₄ = N or D; X₅ = V or I; X₆ = G or S; X₇ = T or N; X₈ = N, T, A or Y; X₉ = V or L; X₁₀ = A, N or S; X₁₁ = S, W, Y or D; X₁₂ = A, T or N; X₁₃ = S or N; X₁₄ = Y, T, N, R or K; X₁₅ = R or L; X₁₆ = Y, H or F; X₁₇ = S or T; X₁₈ = Q or G; X₁₉ = Q or A; X₂₀ = Y, G or W; X₂₁ = N, S or D; X₂₂ = S or T; X₂₃ = Y or L; X₂₄ = P or R; X₂₅ = Y, W or E; X₂₆ = T or A; X₂₇ = N or D; X₂₈ = N; T or A; X₂₉ = S or W; X₃₀ = Y, T or N; X₃₁ = Y or H; X₃₂ = S or T; X₃₃ = N or S; X₃₄ = R or S; X₃₅ = A or G; X₃₆ = Q or S; X₃₇ = D or N; X₃₈ = S or G; X₃₉ = L or V; X₄₀ = N or S; X₄₁ = Y or D; X₄₂ = T or N; X₄₃ = S or N; X₄₄ = R or K; X₄₅ = L or R; X₄₆ = H or F; X₄₇ = Q or G; X₄₈ = Q or A; X₄₉ = G or W; X₅₀ = N or D; X₅₁ = T or S; X₅₂ = P or R; X₅₃ = W or E; X₅₄ = T or A; X₅₅ = K or S; X₅₆ = N or D; X₅₇ = V or I; X₅₈ = G or S; X₅₉ = T or N; X₆₀ = N, A, T or Y; X₆₁= V or L; X₆₂ = A or N; X₆₃ = S, W or Y; X₆₄ = A or T; X₆₅ = Y, N, T or R; X₆₆ = R or L; X₆₇ = Y or H; X₆₈ = S or T; X₆₉ = Y or G; X₇₀ = N or S; X₇₁ = S or T; X₇₂ = Y or L; X₇₃ = Y or W; X₇₄ = R or K; X₇₅ = D or N; X₇₆ = I or V; X₇₇ = S or G; X₇₈ = N or T; X₇₉ = Y or N; X₈₀ = L or V; X₈₁ = N or A; X₈₂ = Y or S; X₈₃ = T or A; X₈₄ = R or Y; X₈₅ = L or R; X₈₆ = H or Y; X₈₇ = G or Y; X₈₈ = T or S; X₈₉ = L or Y; X₉₀ = W or Y.

| | VL CDR1 | SEQ ID No. | VL CDR2 | SEQ ID No. | VL CDR3 | SEQ ID No. |
|---|---|---|---|---|---|---|
| BA61 | **KASQ-NVG-TNVA** | 45 | **SASYRYS** | 55 | **QQYN-SYPYT** | 65 |
| BA3 | **KASQ-NVG-TNVA** | 45 | **SASYRYS** | 55 | **QQYN-SYPYT** | 65 |
| BA5 | **KASQ-NVG-TNVA** | 45 | **SASYRYS** | 55 | **QQYN-SYPYT** | 65 |
| BA29 | **KASQ-NVG-T**A**VA** | 46 | **SAS**N**RY**T | 56 | **QQ**YS-**SYPYT** | 66 |
| BA31 | **KASQ-**D**VG-T**T**VA** | 47 | W**AS**T**R**HT | 57 | **QQY**S-**SYPYT** | 66 |
| BA11 | R**ASQ**-DIS-NYLN | 48 | YT**S**RLH**S** | 58 | **QQ**G**N**-TL**P**W**T** | 67 |
| BA67 | SG**S**SS**N**I**G**NNY**V**S | 49 | DNNK**R**F**S** | 59 | GAWDG**S**LREAV | 68 |
| cons. | | | | | | |
| all | X₁X₂**S**X₃ (S) X₄X₅X₆ (N) X₇X₈X₉X₁₀ | 50 | X₁₁X₁₂X₁₃X₁₄X₁₅ X₁₆X₁₇ | 60 | X₁₈X₁₉X₂₀X₂₁ (G) X₂₂ X₂₃X₂₄X₂₅X₂₆ (V) | 69 |
| BA61+3+ 5+29+31 | **KASQ**X₂₇**VGT**X₂₈**VA** | 51 | X₂₉**AS**X₃₀**R**X₃₁ X₃₂ | 61 | **QQY**X₃₃**SYPYT** | 70 |
| BA61+3+ 5+ | **KASQNVGTNVA** | 45 | **SASYRYS** | 55 | **QQYNSYPYT** | 65 |
| BA11+67 | X₃₄X_{3S}**S**X₃₆ (S) X₃₇I X₃₈ (N) NYX₃₉X₄₀ | 52 | X₄₁X₄₂X₄₃X₄₄X₄₅ X₄₆**S** | 62 | X₄₇X₄₈X₄₉X₅₀ (G) X₅₁ LX₅₂X₅₃X₅₄ (V) | 71 |
| BA61+3+ 5+29+31 +11 | X₅₅**ASQ**X₅₆X₅₇X₅₈X₅₉ X₆₀X₆₁X₆₂ | 53 | X₆₃X₆₄**S**X₆₅X₆₆ X₆₇X₆₈ | 63 | **QQ**X₆₉X₇₀X₇₁X₇₂**P**X₇₃ **T** | 72 |
| BA61+3+ 5+11 | X₇₄**ASQ**X₇₅X₇₆X₇₇X₇₈ X₇₉X₈₀X₈₁ | 54 | X₈₂X₈₃**S**X₈₄X₈₅ X₈₆**S** | 64 | **QQ**X₈₇**N**X₈₈X₈₉**P**X₉₀**T** | 73 |

The amino acid sequences which are designated in claims 11 to 16 refer to the SEQ ID NOs of table 2. For the purpose of clarification of the sequence protocol, the consensus sequences of table 2 which contain amino acids in parentheses (SEQ ID NOs: 50, 52, 69 and 71) are defined in the sequence protocol as follows:
SEQ ID NO: 50 X₁X₂SX₃SX₄X₅X₆NX₇X₈X₉X₁₀
SEQ ID NO: 143 X₁X₂SX₃X₄X₅X₆X₇X₈X₉X₁₀

SEQ ID NO: 52 X₃₄X₃₅SX₃₆SX₃₇IX₃₈NNYX₃₉X₄₀
SEQ ID NO: 144 X₃₄X₃₅SX₃₆X₃₇IX₃₈NYX₃₉X₄₀

SEQ ID NO: 69 X₁₈X₁₉X₂₀X₂₁GX₂₂X₂₃X₂₄X₂₅X₂₆V
SEQ ID NO: 145 X₁₈X₁₉X₂₀X₂₁X₂₂X₂₃X₂₄X₂₅X₂₆

SEQ ID NO: 71 X₄₇K₄₈K₄₉X₅₀GX₅₁LX₅₂X₅₃X₅₄V
SEQ ID NO: 146 X₄₇X₄₈X₄₉X₅₀X₅₁LX₅₂X₅₃X₅₄

Thus, in a preferred embodiment, the antigen-binding polypeptide of the present invention comprises the antigen-binding portion of an antibody heavy chain variable region, which comprises a VH CDR 1, which has the consensus amino acid sequence depicted in SEQ ID NO: 21, more preferably the sequences depicted in SEQ ID NOs: 18, 19 or 20. Other preferred consensus amino acid sequences are depicted in SEQ ID NOs: 22 and 23. Most preferred for VH CDR1 are the individual amino acid sequences depicted in SEQ ID NOs: 18, 19 and 20.

In another preferred embodiment, the antigen-binding polypeptide of the present invention comprises the antigen-binding portion of an antibody heavy chain variable region, which comprises a VH CDR 2, which has the consensus amino acid sequence depicted in SEQ ID NO: 30, more preferably the sequences depicted in SEQ ID NOs: 27, 31 and 33. Other preferred consensus amino acid sequences are depicted in SEQ ID NOs: 32 and 34. Most preferred for VH CDR2 are the individual amino acid sequences depicted in SEQ ID NOs: 24, 25, 26, 27, 28 and 29.

In another preferred embodiment, the antigen-binding polypeptide of the present invention comprises the antigen-binding portion of an antibody heavy chain variable region, which comprises a VH CDR 3, which has the consensus amino acid sequence depicted in SEQ ID NO: 40, more preferably the sequences depicted in SEQ ID NOs: 37, 41 and 43. Other preferred consensus amino acid sequences are depicted in SEQ ID NOs: 42 and 44. Most preferred for VH CDR3 are the individual amino acid sequences depicted in SEQ ID NOs: 35, 36, 37, 38 and 39.

In another preferred embodiment, the antigen-binding polypeptide of the present invention comprises the antigen-binding portion of an antibody light chain variable region, which comprises a VL CDR 1, which has the consensus amino acid sequence depicted in SEQ ID NO: 50, more preferably the sequences depicted in SEQ ID NOs: 48, 49 and 51. Other preferred consensus amino acid sequences are depicted in SEQ ID NOs: 52, 53 and 54. Most preferred for VL CDR1 are the individual amino acid sequences depicted in SEQ ID NOs: 45, 46, 47, 48 and 49.

In another preferred embodiment, the antigen-binding polypeptide of the present invention comprises the antigen-binding portion of an antibody light chain variable region, which comprises a VL CDR 2, which has the consensus amino acid sequence depicted in SEQ ID NO: 60, more preferably the sequences depicted in SEQ ID NOs: 58, 59 and 61. Other preferred consensus amino acid sequences are depicted in SEQ ID NOs: 62, 63 and 64. Most preferred for VL CDR2 are the individual amino acid sequences depicted in SEQ ID NOs: 55, 56, 57, 58 and 59.

In another preferred embodiment, the antigen-binding polypeptide of the present invention comprises the antigen-binding portion of an antibody light chain variable region, which comprises a VL CDR 3, which has the consensus amino acid sequence depicted in SEQ ID NO: 69, more preferably the sequences depicted in SEQ ID NOs: 67, 68 and 70. Other preferred consensus amino acid sequences are depicted in SEQ ID NOs: 71, 72 and 73. Most preferred for VL CDR3 are the individual amino acid sequences depicted in SEQ ID NOs: 65, 66, 67 and 68.

According to the invention, these six different CDRs may be combined with each other in any conceivable way. The CDRs may further be carried by any antibody framework regions. Preferably these framework sequences derive from the mouse, as for example in SEQ ID NOs: 4, 5, 6, 7, 8, 9 or 10.

In another preferred embodiment of the invention, the antigen-binding polynucleotide, which specifically binds to bacterial F4 (K88)-fimbriae, comprises a first polypeptide comprising the antigen-binding portion of the variable region of an antibody heavy chain, a second polypeptide comprising the antigen-binding portion of the variable region of an antibody light chain, and a peptide linker linking said first and said second polypeptide into a single-chain polypeptide.

The term "single-chain polypeptide" or "single-chain antibody" or "single-chain fragment" as used herein means any polypeptide comprising the variable region Fv of a heavy chain, a linking peptide and the variable region Fv of a light chain, resulting in a single chain Fv or "scFv".

In one preferred embodiment, the single-chain antigen-binding polypeptide according to the invention comprises the amino acid sequence of SEQ ID NOs: 4, 5, 6, 7, 8, 9 or 10.

The sequences in SEQ ID NOs: 4, 5, 6, 7, 8, 9 or 10 may further be altered by replacing the linker sequence (here: K L E E G E F S E A R V, SEQ ID NO: 129) with any other desired linker sequence. Further, the respective amino acid sequences of the heavy chain and of the light chain of SEQ ID NOs: 4, 5, 6, 7, 8, 9 and 10 may be combined with each other to form a single-chain antigen-binding polypeptide.

The present invention also refers to multivalent single-chain antigen-binding polypeptides, comprising two or more single-chain polypeptides according to the invention, which specifically bind to bacterial F4 (K88)-fimbriae, and wherein the two or more single-chain polypeptides are linked by a peptide linker. When two single-chain polypeptides are linked by a peptide linker, the polypeptide is called "bivalent".

The peptide linkers linking the polypeptides within a single-chain antigen-binding polypeptide or linking two or more single-chain polypeptides to a multivalent single-chain antigen-binding polypeptide are described in the following.

The peptide linker may be any peptide of any length, preferably in the range of about 1 to 100 amino acids, more preferably in the range of about 12 to 25 amino acids. Preferred linkers of the present invention are e.g. the "yol linker" and the "GS linker". Preferably the peptide linker of the present invention is not susceptible to protease cleavage, particularly to proteases occurring in the gastrointestinal tract of mammals, especially of pigs.

The yol linker used in the present invention has the amino acid sequence
K L E E G E F S E A R V (SEQ ID NO: 129). This linker may also be truncated or elongated at the N terminus and/or at the C terminus by one or more amino acids, as described in the literature. For example, in Schmiedl A. et al. (Protein Eng. 2000 Oct;13(10):725-34) the yol linker is shortened by one amino acid at each terminus compared to the above described yol linker.

In order to improve the stability of the yol linker and to delete the two putative trypsin cleavage sites (lysine and arginine), the yol linker may be modified by exchanging lysine (K) against isoleucine (I), arginine (R) against glycine (G) and valine (V) against cysteine (C). The resulting linker carries the designation "my" (modified yol) and has the amino acid sequence I L E E G E F S E A G C (SEQ ID NO: 130). Any peptide linker carrying the consensus amino acid sequence X₁ L E E G E F S E A X₂ X₃ (SEQ ID NO: 131), wherein X₁ = K or I, X₂ = R or G, and X₃ = V or C, is also preferred in the present invention.

The linker may also be a GS-linker. The GS linker used in the present invention has the amino acid sequence (GGGGS)₂ (SEQ ID NO: 132) and carries the designation "gs". The GS-linker may also have the amino acid sequence (GGGGS)₃, as described in the literature (Huston J.S. Methods Enzymol. 1991;203:46-88). The GS-linker is a preferred linker for the linking of one or more single-chain antigen-binding polypeptides to a multivalent single-chain polypeptide of the present invention.

In one embodiment of the present invention the antigen-binding polypeptide which specifically binds to bacterial F4 (K88) fimbriae is a Fab antibody fragment. The term "Fab" (fragment antigen binding) or "Fab antibody" or "Fab fragment" as used herein means a fragment comprising a complete antibody light chain linked to the N-terminal part of a heavy chain, comprising the variable region (VH) and one constant domain (CH₁). Fab fragments are commercially available, e.g. generated by the company Morphosys, Martinsried/Planegg, Germany.

Other possible antigen-binding polypeptides of the present invention which specifically bind to bacterial F4 (K88) fimbriae are F(ab')₂ fragments (bivalent fragments comprising two linked Fab fragments; Holliger P. and Winter G. Curr. Opin. Biotechnol. 1993 Aug;4(4):446-9) and diabodies (multivalent or multispecific fragments constructed by gene fusion; WO94/13804). The generation of Fab and F(ab')₂ fragments is also described in Liddell, J.E. and I. Weeks (1995) Antibody Technology: A Comprehensive Overview, Bios Scientific: Oxford.

Also preferred in the present invention are non-immunoglobulin polypeptides which specifically bind to bacterial F4 (K88) fimbriae. These may e.g. be ANTICALINS® which are produced by Pieris Proteolab AG (Freising, Germany) or Affilin™ molecules which are produced by Scil Proteins GmbH (Halle, Germany).

ANTICALINS® are derived from lipocalins. Lipocalins are composed of a single polypeptide chain with 160 to 180 amino acid residues. They share a structurally conserved β-barrel supporting four loops at one end, which form the entrance to a binding pocket. The loops exhibit conformational differences between individual lipocalins and give rise to the variety of ligand specificities.

Affilin™ molecules are small non-immunoglobulin proteins based on human cytosolic proteins. Affilin ™ molecules can be selected from two libraries, each of which is based on a different human derived scaffold protein, one of which is gamma crystalline, a human structural eye lens protein. Both human scaffolds are very small, show high temperature stability and are almost resistant to pH changes and denaturing agents. This high stability is mainly due to the expanded beta sheet structure of the proteins.

The antigen-binding polypeptide of the present invention, particularly the antibody fragment and especially the single-chain antibody, which specifically binds to bacterial F4 (K88)-fimbriae, preferably neutralizes the attachment of F4 (K88)-fimbriae to enterocytes.

One example of an *in vitro* assay which assesses the neutralizing effect of the antigen-binding polypeptides of the present invention is the "brush border adhesion test". Brush borders are isolated from the small intestine of mammals, especially of pigs. Under standard conditions, *E.coli* bearing F4 (K88) fimbriae attach to these brush borders. Antigen-binding polypeptides which specifically bind to bacterial F4 (K88) fimbriae are added to this system to measure their neutralizing effect on adhesion. Specific antigen-binding polypeptides protect the intestinal cells from the attaching fimbriae.

In another preferred embodiment, the antigen-binding polypeptide of the present invention has a protective and/or a curative effect on infection of mammals, preferably pigs, with bacteria expressing F4 (K88)-fimbriae.

In order to assess the protective and/or a curative effect, mammals, especially pigs, can be infected with an *E.coli* strain bearing F4 fimbriae. The treatment group receives the antigen-binding polypeptide of the present invention with the feed. The first dose may be applied before or after infection. Thereafter, the animals receive daily doses of the polypeptide, distributed over one or more daily feed applications, for several days. Animals in the control group are infected without polypeptide administration. Monitoring of the clinical symptoms, microbial examination of the feces (which is of high importance for the spread of an infection in a population) and pathological, microbiological and immunochemical analyses (monitoring the presence of the polypeptides in selected organs) give information about the preventative and curative effect of the orally applied antigen-binding polypeptide.

Therefore, another aspect of the present invention is the use of the antigen-binding polypeptide of the present invention, particularly the antibody fragment and especially the single-chain antibody, which specifically binds to F4 (K88)-fimbriae, for the manufacture of a medicament for the treatment and/or prevention of infections with bacteria expressing F4 (K88)-fimbriae. The medicament may be applied to mammals, especially to pigs. The administration preferably occurs orally.

The present invention further relates to the use of the F4 (K88)-binding polypeptides for detecting bacterial F4 (K88) fimbriae, especially for detecting a polypeptide comprising the amino acid sequence of SEQ ID NOs: 1, 2 and/or 3 or a fragment thereof. This detection preferably occurs through binding of the polypeptides of the present invention to the F4 (K88) fimbriae. This binding can take place *in vitro* and/or *in vivo.* For example, the polypeptides of the present invention may be used in research laboratories in cell culture and FACS assays, Western Blot analyses or the like. For the detection purposes, the F4 (K88)-binding polypeptides may be fused to other substances such as epitope tags, fluorescent molecules, enzymes and so on.

It is another object of the invention to provide a composition comprising the antigen-binding polypeptide of the present invention, particularly the antibody fragment and more particularly the single-chain antibody, which specifically binds to F4 (K88)-fimbriae, and an acceptable excipient, carrier, buffer and/or stabilizer.

The antigen-binding polypeptides of the present invention may be degraded rapidly after oral application. Special attention has to be given to the high acidity in the stomach as well as the presence of secretory proteases, particularly in the oral cavity and in the small intestine.

Therefore the excipient, carrier, buffer and/or stabilizer preferably comprises a protein source, preferably BSA, milk powder and/or pea flour. The proteins may be added alone or as a protein mixture. Additionally or alternatively, the excipient, carrier, buffer and/or stabilizer may comprise methyl-cellulose.

Furthermore, the antigen binding peptides may be formulated as acid-resistant micro-particles, or be packed into acid-resistant capsules, preferably gelatine capsules, which are preferably encased with a stabilizing liquid.

The present invention further extends to polynucleotides comprising a nucleotide sequence which encodes an antigen-binding polypeptide according to the invention, which specifically bind to bacterial F4 (K88)-fimbriae. The term "polynucleotide" as used herein is to be understood as meaning a double-stranded or single-stranded nucleic acid molecule, e.g. a DNA, cDNA, genomic DNA, RNA and/or mRNA molecule or the like. The nucleic acid molecule can be present either as the coding strand or as the complementary strand. The polynucleotide may be of a natural source or produced by gene technological or chemical processes and synthesis methods or may have been derived therefrom.

The individual nucleotide sequences and the consensus nucleotide sequences of VH CDR1-3 and VL CDR1-3 of the seven single-chain polypeptides BA61, BA31, BA29, BA3, BA11, BA67 and BA5 are depicted in the following tables 3-8. Nucleotides of the consensus sequences in parentheses are optional. Abbreviations for alternative nucleotides in the nucleotide sequences (underlined) are:
R: G or A
Y: T or C
M: A or C
K: G or T
S: G or C
W: A or T
B: T, G or C
D: A, T or G
H: A, T or C
V: A, G or C
N: any

Table 3: Consensus and individual nucleotide sequences of VH CDR1 of the F4 (K88) binding polypeptides BA61, BA31, BA29, BA3, BA11, BA67 and BA5. The most often occurring common nucleotides are highlighted in bold letters.

| | nucleotide sequence | SEQ ID No. |
|---|---|---|
| BA61 | **AAC TAT GCC ATG TC**T | 74 |
| BA31 | **AAC TAT GCC ATG TC**T | 74 |
| BA29 | **AAC TAT GCC ATG TCC** | 75 |
| BA3 | **AAC TAT GCC ATG TCC** | 75 |
| BA11 | **AAC TAT G**TT **AT**A CA**C** | 76 |
| BA67 | G**A**T **TAT GC**T **ATG** CA**C** | 77 |
| BA5 | G**A**T **TAT GC**T **ATG** CA**C** | 77 |

| consensus: | | |
|---|---|---|
| All | R**A**Y **TAT G**YY **AT**R YMY | 78 |
| BA 61 + 31 + 29 + 3 | **AAC TAT GCC ATG TC**Y | 79 |
| BA 11 + 67 + 5 | R**A**K **TAT G**YT **AT**R CA**C** | 80 |
| BA67+5 | G**A**T **TAT GC**T **ATG** CA**C** | 77 |

Table 4: Consensus and individual nucleotide sequences of VH CDR2 of the F4 (K88) binding polypeptides BA61, BA31, BA29, BA3, BA11, BA67 and BA5. The most often occurring common nucleotides are highlighted in bold letters.

| | nucleotide sequence | SEQ ID No. |
|---|---|---|
| BA61 | **GAA ATT AGT ACT G**G**T GGT AGT** C**AC ACC TAC** TAT CCA GAC ACT GTG ACG GGC | 81 |
| BA31 | **GAA ATT AGT ACT G**C**T GGT AGT** T**AC ACC TAC** TAT CCA GAC ACT GTG ACG GGC | 82 |
| BA29 | **GAA ATT AGT ACT G**C**T GGT AGT** A**AC ACC TAC** TAT CCA GAC ACT GTG ACG GGC | 83 |
| BA3 | **GAA ATT AGT ACT G**C**T GGT AGT** A**AC ACC TAC** TAT CCA GAC ACT GTG ACG GGC | 83 |
| BA11 | T**A**T **ATT A**A**T** C**CT** TAC AA**T** GA**T** TTT **AC**T A**A**A TAC AAT GAG AAG TTC AAA GGC | 84 |
| BA67 | **G**TG **ATT AGT ACT** TAC TC**T** G**GT** AGT **ACA** A**AC** TAC AAC CAG AGC TTT AAG GGC | 85 |
| BA5 | **G**TT **ATT AGT ACT** TAC TC**T** G**GT** AGT **ACA** A**AC** TAC AAC CAG AAG TTT AAG GAC | 86 |
| consensus: | | |
| All | KWD **ATT A**R**T** M**C**T KVY DV**T** RR**T** HDY **AC**H W**A**M **TA**Y MMH S**A**S **A**VB K**T**B **A**MR **G**R**C** | 87 |
| BA 61 + 31 + 29 + 3 | **GAA ATT AGT ACT G**S**T GGT AGT** H**AC ACC TAC** TAT CCA GAC ACT GTG ACG GGC | 88 |
| BA 11 + 67 + 5 | KWK ATT ART M**CT** TAC WM**T** GR**T** WKT **AC**W A**A**M **TA**C AAY S**A**G **A**RS T**T**Y **A**AR **G**R**C** | 89 |
| BA 67 + 5 | **G**TK **ATT AGT ACT** TAC TC**T** G**GT** AGT **AC**A A**AC TA**C AAC C**A**G ARS T**T**T **A**A**G G**R**C** | 90 |

Table 5: Consensus and individual nucleotide sequences of VH CDR3 of the F4 (K88) binding polypeptides BA61, BA31, BA29, BA3, BA11, BA67 and BA5. The most often occurring common nucleotides are highlighted in bold letters.

| | nucleotide sequence | Seq ID No. |
|---|---|---|
| BA61 | **AC**T **GG**T **TAC** --- --- --- --- --- **TTT GAC TAC** | 91 |
| BA31 | **AC**T **GG**T **TAC** --- --- --- --- --- **TTT GAC TAC** | 91 |
| BA29 | **ACG GGG TAC** --- --- --- --- --- **TT**C **GA**T GT**C** | 92 |
| BA3 | **ACG GGG TAC** --- --- --- --- --- **TT**C **GA**T GT**C** | 92 |
| BA11 | GTC **GGG** G**A**A --- --- --- ACC CCC **TTT G**CT **TAC** | 93 |
| BA67 | **ACG G**AC **TAC** CCT TAT TTC TAT GCT A**T**G **GAC TAC** | 94 |
| BA5 | **AC**A AAC **TAC** CCT TAT TTC AAT GGT A**T**G **GAC TAC** | 95 |
| consensus: | | |
| All | RYN RRB K**A**M (CCT TAT TTC)(WMY SSY) W**T**B **G**MY KW**C** | 96 |
| BA 61 + 31 + 29 + 3 | **AC**K **GG**K **TAC** --- --- --- --- --- **TT**Y **GA**Y KW**C** | 97 |
| BA 11 + 67 + 5 | RYV RRS K**A**M (CCT TAT TTC) WMY SSY W**T**K GMY **TAC** | 98 |
| BA 67 + 5 | **AC**R RAC **TAC** CCT TAT TTC WAT GST A**T**G **GAC TAC** | 99 |

Table 6: Consensus and individual nucleotide sequences of VL CDR1 of the F4 (K88) binding polypeptides BA61, BA31, BA29, BA3, BA11, BA67 and BA5. The most often occurring common nucleotides are highlighted in bold letters.

| | nucleotide sequence | SEQ ID No. |
|---|---|---|
| BA61 | **AAG GCC AGT CAG --- AAT GTG GGT --- ACT AAT GTA GCC** | 100 |
| BA3 | **AAG GCC AGT CAG --- AAT GTG GGT --- ACT AAT GTA GCC** | 100 |
| BA5 | **AAG GCC AGT CAG --- AAT GTG GGT --- ACT AAT GTA GCC** | 100 |
| BA29 | **AAG GCC AGT CAG --- AAT GTG GGT --- ACT** GC**T GTA GCC** | 101 |
| BA31 | **AAG GCC AGT CAG ---** G**AT GTG GGT --- ACT A**C**T GTA GCC** | 102 |
| BA11 | **A**G**G GC**A **AGT CAG ---** G**A**C A**T**T A**G**C **--- A**A**T** T**AT** T**TA** AA**C** | 103 |
| BA67 | TCT **G**GA **AG**C AGC TCC **AA**C A**T**T **GG**A AAT **A**A**T** T**AT GT**C T**CC** | 104 |
| consensus: | | |
| all | WVK **G**SM **AG**Y MRS (TCC) R**A**Y R**T**K R**G**H (AAT) **A**M**T** DM**T** K**T**M DM**C** | 105 |
| BA61+3+5+29 +31 | **AAG GCC AGT CAG** --- R**AT GTG GGT** --- **ACT** RM**T GTA GCC** | 106 |
| BA61+3+5 | **AAG GCC AGT CAG** --- **AAT GTG GGT** --- **ACT AAT GTA GCC** | 100 |
| BA11+67 | WSK **G**SA **AG**Y MRS (TCC) R**A**C A**T**T R**G**M (AAT) **A**AT T**AT** K**T**M WM**C** | 107 |
| BA61+3+5+29 +31+11 | **A**R**G GC**M **AGT CAG** --- R**A**Y R**T**K R**G**Y --- **A**M**T** DM**T** K**TA** RM**C** | 108 |
| BA61+3+5+11 | **A**R**G GC**M **AGT CAG ---** R**A**Y R**T**K R**G**Y **--- A**M**T** W**AT** K**TA** RM**C** | 109 |

Table 7: Consensus and individual nucleotide sequences of VL CDR2 of the F4 (K88) binding polypeptides BA61, BA31, BA29, BA3, BA11, BA67 and BA5. The most often occurring common nucleotides are highlighted in bold letters.

| | nucleotide sequence | SEQ ID No. |
|---|---|---|
| BA61 | **TCG GCA TCC TAC CGG TAC AGT** | 110 |
| BA3 | **TCG GCA TCC TAC CGG TAC AGT** | 110 |
| BA5 | **TCG GCA TCC TAC CGG TAC AGT** | 110 |
| BA29 | **TCG GCA TCC** A**A**T **CGG TAC A**C**T** | 111 |
| BA31 | **T**G**G GCA TCC** AC**C CGG** C**AC A**C**T** | 112 |
| BA11 | **T**AC A**CA TC**A AGA TTA C**AC** TCA | 113 |
| BA67 | GAC AAT AAT A**A**G **CG**A **T**T**C** TCA | 114 |
| consensus: | | |
| all | KVS RMW WMH WVN YKR YWC WSW | 115 |
| BA61+3+5+29+31 | **T**S**G GCA TCC** WMY **CGG** Y**AC A**S**T** | 116 |
| BA61+3+5 | **TCG GCA TCC TAC CGG TAC AGT** | 110 |
| BA11+67 | KAC AMW WMW ARR YKA YW**C** TCA | 117 |
| BA61+3+5+29+31+11 | **T**VS R**CA TC**M WVH YKR Y**AC** WSW | 118 |
| BA61+3+5+11 | **T**MS R**CA TC**M WRM YKR Y**AC** WSW | 119 |

Table 8: Consensus and individual nucleotide sequences of VL CDR3 of the F4 (K88) binding polypeptides BA61, BA31, BA29, BA3, BA11, BA67 and BA5. The most often occurring common nucleotides are highlighted in bold letters.

| | nucleotide sequence | SEQ ID No. |
|---|---|---|
| BA61 | **CAG CAA TAT AAC --- AGC TAT CCT TAC ACG** | 120 |
| BA3 | **CAG CAA TAT AAC --- AGC TAT CCT TAC ACG** | 120 |
| BA5 | **CAG CAA TAT AAC --- AGC TAT CCT TAC ACG** | 120 |
| BA29 | **CAG CAA TAT A**G**C --- AGC TAT CC**G **TAC ACG** | 121 |
| BA31 | **CAG CAA TAT A**G**C --- AGC TAT CC**G **TAC ACG** | 121 |
| BA11 | **CA**A **CA**G GG**T AA**T **--- A**CG CT**T CC**G **T**GG **ACG** | 122 |
| BA67 | GGA GC**A T**GG G**A**T GGC **AGC** CTG **C**G**T** G**A**A G**CG** GTA | 123 |
| consensus: | | |
| all | SRR SMR KRK RRY (GGC) **A**SS YWK **C**SK KRV R**CG** (GTA) | 124 |
| BA61+3+5+29+31 | **CAG CAA TAT A**RC --- **AGC TAT CC**K **TAC ACG** | 125 |
| BA61+3+5 | **CAG CAA TAT AAC** --- **AGC TAT CCT TAC ACG** | 120 |
| BA11+67 | SRA SMR KGK R**A**T (GGC) **A**SS CTK **C**SK KRR R**CG** (GTA) | 126 |
| BA61+3+5+29+31+11 | **CA**R **CA**R KR**T A**RY --- **A**SS YW**T CC**K **T**RS **ACG** | 127 |
| BA61+3+5+11 | **CA**R **CA**R KR**T AA**Y --- **A**SS YW**T CC**K **T**RS **ACG** | 128 |

Thus, in a preferred embodiment, the polynucleotide of the present invention comprises the nucleotide sequence encoding the antigen-binding portion of an antibody heavy chain variable region, which comprises a VH CDR 1, which is encoded by the nucleotide sequence depicted in SEQ ID NO: 78, more preferably by the sequences depicted in SEQ ID NOs: 76, 77 and 79. Another preferred consensus nucleotide sequence is depicted in SEQ ID NO: 80. Most preferred for encoding VH CDR1 are the individual nucleotide sequences depicted in SEQ ID NOs: 74-77. Also preferred in the present invention is a polynucleotide which comprises a nucleotide sequence encoding the VH CDR1 depicted in the amino acid sequences SEQ ID NOs: 18-23, preferably 18-20.

In another preferred embodiment, the polynucleotide of the present invention comprises the nucleotide sequence encoding the antigen-binding portion of an antibody heavy chain variable region, which comprises a VH CDR 2, which is encoded by the nucleotide sequence depicted in SEQ ID NO: 87, more preferably by the sequences depicted in SEQ ID NOs: 84, 88 and 90. Another preferred consensus nucleotide sequence is depicted in SEQ ID NO: 89. Most preferred for VH CDR2 are the individual nucleotide sequences depicted in SEQ ID NOs: 81-86. Also preferred in the present invention is a polynucleotide which comprises a nucleotide sequence encoding the VH CDR2 depicted in the amino acid sequences SEQ ID NOs: 24-34, preferably 24-29.

In another preferred embodiment, the polynucleotide of the present invention comprises the nucleotide sequence encoding the antigen-binding portion of an antibody heavy chain variable region, which comprises a VH CDR 3, which is encoded by the nucleotide sequence depicted in SEQ ID NO: 96, more preferably by the sequences depicted in SEQ ID NOs: 93, 97 and 99. Another preferred consensus nucleotide sequence is depicted in SEQ ID NO: 98. Most preferred for VH CDR3 are the individual nucleotide sequences depicted in SEQ ID NOs: 91-95. Also preferred in the present invention is a polynucleotide which comprises a nucleotide sequence encoding the VH CDR3 depicted in the amino acid sequences SEQ ID NOs: 35-44, preferably 35-39.

In another preferred embodiment, the polynucleotide of the present invention comprises the nucleotide sequence encoding the antigen-binding portion of an antibody heavy chain variable region, which comprises a VL CDR 1, which is encoded by the nucleotide sequence depicted in SEQ ID NO: 105, more preferably by the sequences depicted in SEQ ID NOs: 103, 104 and 106. Other preferred consensus nucleotide sequences are depicted in SEQ ID NOs: 107-109. Most preferred for VL CDR1 are the individual nucleotide sequences depicted in SEQ ID NOs: 100-104. Also preferred in the present invention is a polynucleotide which comprises a nucleotide sequence encoding the VL CDR1 depicted in the amino acid sequences SEQ ID NOs: 45-54, preferably 45-49.

In another preferred embodiment, the polynucleotide of the present invention comprises the nucleotide sequence encoding the antigen-binding portion of an antibody heavy chain variable region, which comprises a VL CDR 2, which is encoded by the nucleotide sequence depicted in SEQ ID NO: 115, more preferably by the sequences depicted in SEQ ID NOs: 113, 114 and 116. Other preferred consensus nucleotide sequences are depicted in SEQ ID NOs: 117-119. Most preferred for VL CDR2 are the individual nucleotide sequences depicted in SEQ ID NOs: 110-114. Also preferred in the present invention is a polynucleotide which comprises a nucleotide sequence encoding the VL CDR1 depicted in the amino acid sequences SEQ ID NOs: 55-64, preferably 55-59.

In another preferred embodiment, the polynucleotide of the present invention comprises the nucleotide sequence encoding the antigen-binding portion of an antibody heavy chain variable region, which comprises a VL CDR 3, which is encoded by the nucleotide sequence depicted in SEQ ID NO: 124, more preferably by the sequences depicted in SEQ ID NOs: 122, 123 and 125. Other preferred consensus nucleotide sequences are depicted in SEQ ID NOs: 126-128. Most preferred for VL CDR3 are the individual nucleotide sequences depicted in SEQ ID NOs: 120-123. Also preferred in the present invention is a polynucleotide which comprises a nucleotide sequence encoding the VL CDR1 depicted in the amino acid sequences SEQ ID NOs: 65-73, preferably 65-68.

The polynucleotides may also comprise sequences which hybridize to a complementary strand of the above mentioned nucleotide sequences or are a degenerate of the above mentioned nucleotide sequences.

In another preferred embodiment, the polynucleotide comprising a nucleotide sequence which encodes an antigen-binding polypeptide according to the invention, which specifically binds to bacterial F4 (K88)-fimbriae, is selected from the group consisting of
a) polynucleotides comprising a nucleotide sequence which encodes the amino acid sequence identified in SEQ ID NOs: 4, 5, 6, 7, 8, 9 or 10,
b) polynucleotides comprising the nucleotide sequence identified in SEQ ID NOs: 11, 12, 13, 14, 15, 16 or 17,
c) polynucleotides comprising a nucleotide sequence which hybridizes to a complementary strand of the nucleotide sequence of a) or b),
d) polynucleotides comprising a nucleotide sequence which is degenerate to a nucleotide sequence of c),
e) polynucleotides which represent a derivative, analogue, part or fragment of a nucleotide sequence of a), b), c) or d).

The terms "to hybridize" or "hybridization" describe the process by which a single-stranded polynucleotide enters into base-pairing with a complementary polynucleotide strand. In the context of the present invention the term "hybridization" means a hybridization under conventional hybridization conditions, preferably under stringent conditions, for example as described in Sambrook et al. (1989), Molecular Cloning: A Laboratory Manual, 2. Ed., Cold Spring Harbour Laboratory Press, Cold Spring Harbour, NY.

Suitable stringent conditions include salt solutions of about 0.9 molar at temperatures of 35°C to 65°C. Stringent hybridization conditions preferably comprise the following conditions:

| | | | |
|---|---|---|---|
| Hybridization buffer: | 7% SDS | | |
| | 250 mM NaCl | | |
| | 250 mM K-phosphate buffer pH 7.0 | | |
| | 1 mM EDTA | | |
| Hybridization temperature: | 58°C to 60°C | | |
| Hybridization time: | overnight | | |
| Washing buffer: | I | 2 x SSC | 0.1% SDS |
| | II | 0.2 x SSC | 0.1% SDS |
| Washing temperature and time: | each 2 x 30 min at 55°C to 60°C | | |

The degeneration of the genetic code offers one skilled in the art among other things the possibility of adapting the nucleotide sequence to the codon preference of the target host cell, thereby optimising the expression of the desired antigen-binding polypeptide of the present invention.

The above-mentioned polynucleotides also comprise fragments, derivatives, analogues or parts of the polynucleotide sequences described above, which code for a polypeptide, particularly an antibody fragment, and especially a single-chain antibody, having the biological characteristics of a polypeptide which specifically binds to bacterial F4 (K88)-fimbriae. The fragments, derivatives, analogues or parts may also be both naturally occurring variations or mutations, wherein these mutations may have occurred naturally or have been introduced e.g. by targeted mutagenesis. Moreover, the variations can further comprise synthetic sequences.

The polypeptide being encoded by the fragments, derivatives, analogues or parts of the polynucleotide sequences described above is preferably characterized by "conservative" amino acid substitutions, for instance substitution of one hydrophobic residue for another or the substitution of one polar residue for another. At certain positions non-conservative substitutions are allowable.

The term "fragments" is to be understood as parts of the polynucleotide sequence that are sufficiently long to encode one of the described polypeptides. The term "derivative" in this context means that the sequences differ from the polynucleotide sequences described above at one or several position(s), but have a high degree of homology to these sequences. Homology here means a sequence identity of at least 40%, particularly an identity of at least 60% or 70%, preferably of at least 80%, 82%, 84%, 86% or 88%, and particularly preferably of at least 90%, 92%, 94%, 96% or 98%. Variations from the nucleotide sequences described above may be caused, for example, by deletion, substitution, insertion or recombination.

The proteins encoded by these polynucleotide sequences show a sequence identity to the amino acid sequences identified in SEQ ID NOs: 4, 5, 6, 7, 8, 9 or 10, respectively, of at least 40%, particularly an identity of at least 60% or 70%, preferably of at least 80%, 82%, 84%, 86% or 88%, and particularly preferably of at least 90%, 92%, 94%, 96% or 98%. The differences to the above described polynucleotide sequences may be the result of, for example, deletion, substitution, insertion or recombination.

In order to determine the percentage of homology (= identity) between two amino acid or nucleotide sequences, the two sequences are aligned, and the amino acids or nucleotides at each position are compared. If one position within the sequences is occupied by the same amino acid or the same nucleotide, then the molecules at this position are homologous (= identical). The percentage of homology between the two sequences is a function of the number of common positions that are identical (i.e. homology = number of identical positions per total number of positions x 100).

The homology is calculated over the total amino acid or nucleotide sequence area. In order to compare different sequences a variety of programs based on different algorithms are available to the person skilled in the art. The algorithms of Needleman and Wunsch or Smith and Waterman provide especially reliable results. For the sequence alignments and comparisons, the programs "PileUp" (J. Mol. Evolution., 25, 351-360, 1987, Higgins et al., CABIOS, 5 1989: 151―153) or "Gap" and "BestFit" [Needleman and Wunsch (J. Mol. Biol. 48; 443-453 (1970) and Smith and Waterman (Adv. Appl. Math. 2; 482-489 (1981)], which are enclosed in the GCG Software-Packet [Genetics Computer Group, 575 Science Drive, Madison, Wisconsin, USA 53711 (1991)], can be used. The sequence homology values mentioned above as percentages can be determined by means of the "Gap" program over the total sequence area with the following adjustments: Gap Weight: 50, Length Weight: 3, Average Match: 10.000 und Average Mismatch: 0.000. These adjustments can be used as standard adjustments for sequence homology analyses.

The polynucleotides comprising the nucleotide sequence identified in SEQ ID NOs: 11, 12, 13, 14, 15, 16 or 17 may be altered by replacing the linker encoding nucleotide sequence (here: AAG CTT GAA GAA GGT GAA TTT TCA GAA GCA CGC GT, SEQ ID NO: 133) with any other desired linker encoding nucleotide sequence. Further, the nucleotide sequences encoding the heavy chain and the nucleotide sequences encoding the light chain of SEQ ID NOs: 11, 12, 13, 14, 15, 16 or 17 may be combined with one another in any conceivable way.

Preferably, the polynucleotide of the present invention comprising a nucleotide sequence encoding the antigen-binding polypeptide, which specifically binds to bacterial F4 (K88)-fimbriae, is under control of regulatory sequences which provide for a specific transcription. This can be achieved by conventional cloning methods (e.g.

Sambrook et al. 1989, Molecular Cloning: A Laboratory Manual, 2. Ed., Cold Spring Harbour Laboratory Press, Cold Spring Harbour, NY). Thus, the present invention further provides a recombinant polynucleotide molecule, which comprises
a) a promoter region,
b) a polynucleotide according to the invention as described above, which is operatively linked to the promoter region, and
c) optionally, regulatory sequences operatively linked thereto, which may act as transcription, termination and/or polyadenylation signals, enhancer sequences and/or sequences coding for leader signals.

Using routine molecular biological techniques (see for example Sambrook *et al.,* vide supra) it is possible to prepare and to produce the desired constructs for the transformation of any prokaryotic or eukaryotic cell. Methods for cloning, mutagenesis, sequence analysis, restriction analysis and other biochemical/molecular biological methods are well known to the person skilled in the art and are conventionally used for gene technological manipulation.

Thus, not only may suitable polynucleotide constructs containing the desired fusion of a promoter sequence and an antigen-binding polypeptide sequence of the present invention and optionally further regulatory sequences be produced, but rather the person skilled in the art may further, if desired, introduce various mutations, deletions, fusions or insertions of natural or artificial nucleotide sequences into the nucleotide sequence encoding the antigen-binding polypeptide according to the invention using routine techniques. For the fusion of the constructs with each other adapters or linkers may be attached to the constructs where necessary. Additionally, appropriate restriction sites may be provided or removed using enzymatic and other manipulation techniques. This may result in the synthesis of a protein with altered biological properties, e.g. stronger binding properties (affinity, avidity, time period) or higher stability, e.g. temperature stability, pH stability and/or stability against protease cleavage, or resulting in the synthesis of proteins with altered expression properties, e.g. at the transcription and/or translation level, or with altered purification capacities.

The present invention also refers to vectors comprising the polynucleotide which encodes the antigen-binding polypeptide of the present invention, particularly the antibody fragment and more particularly the single-chain antibody, which specifically binds to bacterial F4 (K88)-fimbriae. The term "vector" describes a polynucleotide which is used for introducing exogenous polynucleotides into host cells. A vector contains a nucleotide sequence which encodes one or more polypeptides. Preferably the vector of the present invention is a phage display vector, a bacterial expression vector, a yeast expression vector, a fungus expression vector, an algae expression vector or a plant expression vector. Vectors which are able to control the expression of the genes which they contain are termed "expression vectors".

In order to prepare the introduction of foreign genes in higher plants a variety of vectors are available which contain replicating signals for *E. coli* and a marker gene for selecting transformed bacterial cells. Examples for such vectors are pBR322, pUC series, M13mp series, pACYC184, pBlueSfi and the like. The desired sequence may be introduced in the vector at a suitable restriction site. The resulting plasmid is then used for the transformation of *E.coli* cells. Transformed *E.coli* cells are cultivated in a suitable medium and then harvested and lysed, and the plasmid is recovered. In order to characterize the produced plasmid DNA in general restriction analysis, gel electrophoresis and further biochemical and molecular biological methods are used as analytic method. After each manipulation step the plasmid DNA may be cleaved and the obtained DNA fragments may be linked to other DNA sequences.

Several known techniques are available for introducing DNA in a plant host cell, and the person skilled in the art will not have any difficulties in selecting a suitable method. These techniques comprise the transformation of plant cells with T DNA by use of *Agrobacterium tumefaciens* or *Agrobacterium rhizogenes* as the transforming agent, the fusion of protoplasts, the direct gene transfer of isolated DNA into protoplasts, the electroporation of DNA, the introduction of DNA by means of the biolistic method as well as further possibilities.

During the injection and electroporation of DNA into plant cells no specific requirements for the used plasmids are necessary per se. The same is true for the direct gene transfer. Plain plasmids such as pUC and pBlueScript derivatives may be used. The presence of a selectable marker gene is necessary if entire plants are to be regenerated from such transformed cells. The person skilled in the art is familiar with these gene selection markers and will not have any problems in selecting a suitable one.

Further DNA sequences may be required depending on the introduction method for desired genes into the plant cell. If, for example, the Ti or Ri plasmid is used for the transformation of the plant cell, at least the right border, however more often both, the right and the left border of the T DNA in the Ti or Ri plasmid, has to be linked as flanking region to the genes to be introduced.

If agrobacteria are used for the transformation, the DNA to be introduced has to be cloned into special plasmids, either into an intermediate or into a binary vector. Intermediate vectors may be integrated into the Ti or Ri plasmid of the agrobacteria by homologous recombination via sequences which are homologous to sequences in the T DNA. This also contains the *vir* region which is required for T DNA transfer. Intermediate vectors are not able to replicate in agrobacteria. With the aid of a helper plasmid the intermediate vector may be transferred to *Agrobacterium tumefaciens* (conjugation). Binary vectors are able to replicate in *E.coli* as well as in agrobacteria. They contain a selection marker gene, and a linker or polylinker framed by the right and left T DNA border region. They may be transformed directly into agrobacteria. The agrobacterial host cell should contain a plasmid with a *vir* region. The *vir* region is required for the transfer of the T DNA into the plant cell. Additional T DNA may be present. Such a transformed agrobacterial cell is used for the transformation of plant cells.

The use of T DNA for the transformation of plant cells has been studied intensively, and has been sufficiently described in generally known reviews and plant transformation manuals.

For transfer of the DNA into the plant cell, plant explants may be cultivated for this purpose together with *Agrobacterium tumefaciens or Agrobacterium rhizogenes.* From the infected plant material (e.g. leaf pieces, stem segments, roots, but also protoplasts or suspension-cultivated plant cells) whole plants may be regenerated in a suitable medium which may contain antibiotics or biocides for selection of transformed cells. Regeneration of plants may take place according to conventional regeneration methods by use of known growth media. The resulting plants may be analysed for the presence of the introduced DNA. Other possibilities for the introduction of foreign DNA by use of biolistic methods or protoplast transformation are well known, and as have been described extensively.

Once the introduced DNA has been integrated into the plant cell genome it is generally stable there and is maintained in the progeny of the originally transformed cell as well. Usually it contains a selection marker which allows the selection of transformed cells from those lacking the introduced DNA. This selection marker endows the transformed plant cells with resistance to a biocide or an antibiotic, such as kanamycin, G418, bleomycin, hygromycin, methotrexate, glyphosate, streptomycin, sulfonylurea, gentamycin or phosphinotricin and others. But also nutritive markers and screening markers (such as GFP, green fluorescent protein) are being used as alternatives to antibiotics resistance markers.It is also possible to perform this selection procedure without any selection marker, however this requires a quite high screening expenditure.

The resulting plants may be cultivated normally, and may be crossbred with plants having the same transformed hereditary disposition or other predispositions. The resulting hybrids will have the pertinent phenotype characteristics. Seeds may be obtained from the plant cells.

Two or more generations should be generated to ensure that the phenotypic feature is stably maintained and inherited. Additionally, seeds should be harvested to verify the maintenance of the respective phenotype or other features.

In a preferred embodiment, the vector comprising the polynucleotide which encodes the antigen-binding polypeptide of the present invention, particularly the antibody fragment and more particularly the single-chain antibody, which specifically binds to bacterial F4 (K88)-fimbriae, contains specific regulatory elements, either constitutive or inducible (which may be either by biotic or abiotic stimuli), especially promoters, specific for the expression of the cloned construct in the prokaryotic or eukaryotic host cell, e.g. in bacteria, yeast or plant cells. For the expression of the polypeptides of the present invention in a plant cell or a microorganism cell, in principal any promoter can be considered which is active in the respective host cells. The promoter may be homologous or heterologous to the cell to be transformed.

For plant expression, the promoter is preferably tissue specific, but it may also be development-specific. The promoter may e.g. be active in tissues that are used as fodder such as seed, leaf and fruit tissues. The most preferred promoters for plant expression are seed specific promoters.

Useful promoters for seed specific expression are the USB promoter, preferably the long (Zakharov et al. 2004, J Exp Bot., Jul;55(402):1463-71. Epub 2004 Jun 4) or the short USB promoter (Bäumlein et al. 1991, Mol. Gen. Genet. 225:459-467). Other promoters which can be used in the present invention are the NOS-promoter, the Vicilin promoter, the Phaseolin promoter, the Actin promoter, the Arcelin promoter, the Hordein promoter (Brandt et al. 1985, Carlsberg Res. Commun. 50:333-345) and the Napin promoter, the ACP promoter as well as the FatB3- and FatB4 promoters, the 35S RNA promoter of the Cauliflower Mosaic Virus, the Ubiquitin promoter from maize, or the LeB4 promoter. The following publication summarizes most of the useful promoters: Zakharov et al. 2004, J Exp Bot., Jul;55(402):1463-71. Epub 2004 Jun 4.

Leader sequences may also be introduced into the vector, e.g. in order to achieve secretion of the polypeptides of the present invention into the medium. For example, the amino acid sequence "EAYA" is a cleavage site at the C terminus of a leader sequence. The "KDEL" sequence at the C terminus of a polypeptide sequence is an endoplasmatic reticulum (ER) retention signal in plants.

Useful promoters for the expression in microorganisms are e.g. the Lac Operon/promoter, the T3/T7 promoter, and the AOX promoter.

These promoters are well known to a person skilled in the art of molecular biology. In any case the skilled person can find suitable promoters in the literature, e.g. in relevant scientific journals and gene databases, or can isolate them from any desired organism using standard methods.

Another aspect of the present invention is to provide prokaryotic or eukaryotic host cells which comprise a polynucleotide encoding an antigen-binding polypeptide of the present invention or a vector comprising said polynucleotide. Preferably, the host cells are stably or transiently transformed with the above-described vectors of the present invention. Still another aspect of the present invention is to provide transgenic plants or parts thereof as well as microorganisms containing an above-described polynucleotide or an above-described vector of the present invention.

Preferably, the host cells or the plants or plant parts or microorganisms of the present invention express and synthesize the antigen-binding polypeptide of the present invention.

These host cells may be any prokaryotic or eukaryotic cells, preferably microorganismic cells or plant cells, more preferably bacterial, yeast, fungus, algae and plant cells. Most preferred among the microorganismic cells are *Escherichia coli* cells, Streptomyces cells, *Pichia pastoris* cells, *Schizosaccharomyces* cells, especially *Schizosaccharomyces pombe* cells, Aspergillus cells, and Chlamydomonas cells.

The plant cells and plants of the present invention may be in principle any plant cells or plants. Preferably, it is a monocotyledonous or dicotyledonous crop plant and more preferably a fodder plant. The most preferred plants are a *Nicotiana spec.,* especially *Nicotiana benthamiana* and *Nicotiana tabacum, Pisum sativum, Hordeum vulgare, Zea mays, Cucumis sativus* and *Linum usitatissimum* and rapeseed. Other plants which may be used are e.g. flax, poppy, olive, cocoa, barley, wheat, almond, sesame, mustard, castor oil plant, sunflower, soybean, peanut, coconut, turnip seed, cotton, cucumber, tobacco and oil palm trees.

The invention also relates to propagation material and agricultural products of the plants according to the invention, for example fruits, seeds, tubers, rootstocks, seedlings, cuttings and the like.

Further, the invention relates to the use of the plants, plant parts or microorganisms of the present invention as fodder for mammals, especially for pigs.

Another object of the invention is to provide a method of producing an antigen-binding polypeptide of the present invention, particularly an antibody fragment and more particularly a single-chain antibody, which specifically binds to F4 (K88)-fimbriae, comprising:
- providing an expression vector which comprises a polynucleotide encoding said antigen-binding polypeptide, and
- expressing said vector in a prokaryotic or eukaryotic host cell.

The expression vector, the polynucleotide and the host cell used for this method are described above.

### EXAMPLES

The following examples are intended to illustrate the present invention and are in no way to be understood as limiting.

### Example 1: Generation of a phage display library

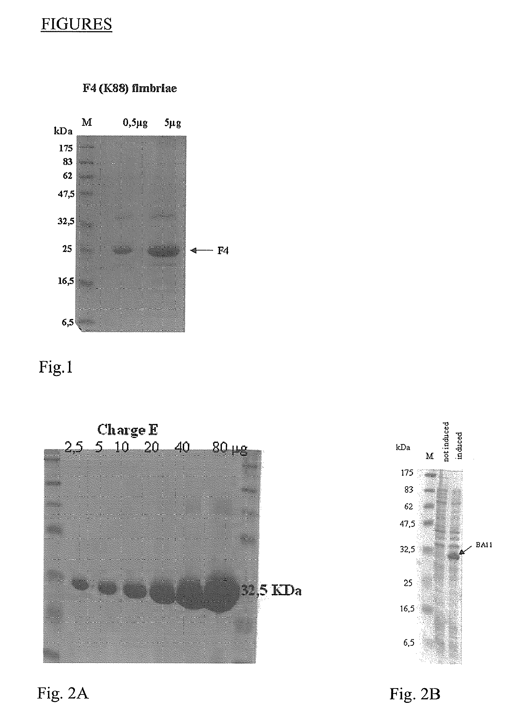

Fimbriae were isolated from an *E.coli* strain bearing F4 (K88) fimbriae, purified and separated on an SDS gel (fig. 1; molecular weight: 27,6 kDa). Mice were immunized with this antigen, Blood samples showed high antibody titers in the antigen ELISA. The spleens of the mice were prepared and RNA isolated therefrom. After mRNA purification single stranded DNA was prepared. DNA quality was tested by PCR amplification of the immunoglobulin genes. An aliquot of the cDNA was cloned into a phage display vector. The phage display library contained recombinant antibody fragments (scFv = single chain fragment variable).

For library screening the F4 antigen was immobilized on Maxisorp immunoplates and the phage display library was cycled three times for binding selection. Wells were blocked with BSA. Clones were selected randomly and assayed using a phage ELISA to compare binding to target antigen (F4 fimbriae) and several non-relevant proteins for preventing non-specific binding. Positive clones (only binding to F4) were further analyzed by restriction analysis, sequencing, western blot and ELISA. The following clones were isolated (table 9):

Table 9: scFv antibodies obtained from a screening with F4-fimbriae. "Screened clones" are the clones which were analyzed by ELISA (576). 133 gave an ELISA signal up to 0,8 and 319 an ELISA signal up to 0,2. From the 576 clones analyzed by ELISA only 96 were further analyzed (protein expression, western blot, DNA extraction, restriction analysis). Unique scFv are scFv which have the same amino acid sequence. Max. 3 amino acids (10 amino acids) are different.

| antigen | nomenclature | screened clones | positive clones ELISA >0,2 (>0,8) | analyzed clones | unique scFv > 3 amino acids (> 10 amino acids) | in cell culture positive tested medium (strong) |
|---|---|---|---|---|---|---|
| F4 (K88) *E. coli* | BAxx | 576 | 319 (133) | 96 | 35 (25) | 6 (3) |

Three of the isolated scFv antibodies against F4-fimbriae showed a strong neutralizing effect (BA61, BA3 and BA11). Among the antibodies tested "medium" are BA5, BA29, BA31 and BA67.

For the ELISA assay, Maxisorp-plates (Nunc) were coated over night at 4°C with 0,5 µg / well F4 antigen (in PBS pH 7,4). Plates were washed three times with PBS and blocked two hours at room temperature (RT). After three additional washes the BA11 antibody (blocked for 30min with 6% BSA-PBS at RT) was added for two hours. Plates were washed once with PBS/0,05%Tween and four times with PBS before adding the primary anti-His antibody (Sigma H1029) diluted 1:3000 in 2 % BSA-PBS for one hour at RT. After washing once with PBS/0,05%Tween and four times with PBS the secondary anti-mouse-HRP-conjugated antibody (Sigma A4312) diluted 1:8000 in 2% BSA-PBS was incubated for one hour at RT. Plates were washed again once with PBS/0,05%Tween and four times with PBS before TMB/H₂O₂ was added. After 30min the reaction was stopped by adding 1M sulfuric acid and the OD₄₅₀ₙₘ was measured. The cell culture assay is described in example 3.

### Example 2: Expression of scFv antibodies

As expression systems (production systems) for the scFv antibodies *E.coli* and *Pichia pastoris* were used. Antibody binding peptides were cloned into specific expression vectors.

The production of the scFv BA11 in *Pichia pastoris* was optimized starting from a 20ml expression.

| | |
|---|---|
| Expression rate: | 25-50 mg/l culture medium |
| Purification loss: | < 15% |
| Purity: | > 95% |
| Time needed: | maximum two weeks for pure protein |
| Storage stability: | lyophilized and stored at 4°C for several months (most probably longer) |

Fig. 2A shows a 12% SDS-gel with a dilution series of scFv-BA11 produced in *Pichia pastoris.*

BA11 expression in *E.coli*: The BA11 was cloned into the pET16b vector (Novagen) and transformed into BL21 cells (Stratagene). Cells were cultured until OD₆₀₀ = 0,6 at 37°C in LB-medium. After induction with 100µM IPTG cells were grown for one hour at 37°C in LB-medium resulting in the expression of the BA11 construct. Cells were harvested by centrifugation and analyzed on a 12% SDS-gel (fig. 2B).

### Example 3: In vitro testing of antibodies (brush-border adhesion and adhesion-inhibition assay)

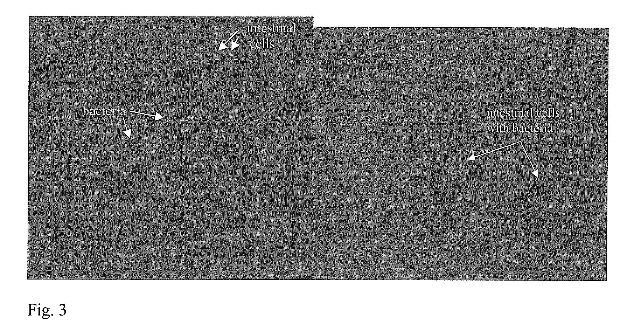

A brush border adhesion test was established to assess the neutralizing effect of the isolated scFv antibodies. Brush borders were isolated from the small intestine of pigs. Under standard conditions *E.coli* bearing F4 fimbriae attach to these brush borders. Antibodies were added to this system to measure their neutralizing effect on adhesion (Fig. 3). The strongest neutralization effect was shown by the scFv antibodies BA11, BA61 and BA3. None of the negative controls or antibodies against other pathogens showed an inhibitory effect.

An *in vitro* brush-border adhesion assay was performed as described by Van den Broeck W. et al. (Vaccine. 1999 Apr 9;17(15-16):2020-9) and Dean-Nystrom E.A. et al. (Vet Microbiol. 1993 Oct;37(1-2):101-14). Subsequently after brush border isolation 4x10⁸ F4ac*⁺E.coli* were added to an average of 100 villi in 60µl PBS buffer in a microtiter plate (Nunc) and incubated for 2 hours at room temperature. Adhesion was examined by phase contrast microscopy at magnification x 600.

For adhesion-inhibition assay bacteria (4x10⁸ F4ac⁺) were incubated 20min at room temperature with scFv antibody (serial dilutions, starting at 0,2mg/ml) before adding the brush-borders (100/60µl).

Table 10: ScFv antibodies tested in the brush border adhesion assay. BB= antibodies against F5-fimbriae, BC= antibodies against F41-fimbriae.

| scFv | neutralization | | controls | neutralization |
|---|---|---|---|---|
| BA 11 | strong | | no scFv | none |
| BA 61 | strong | | boiled BA 3 | none |
| BA 3 | strong | | boiled BA 61 | none |
| BA 5 | medium | | BB 2 | none |
| BA 29 | medium | | BB 15 | none |
| BA 31 | medium | | BC 14 | none |
| BA 67 | medium | | | none |

### Example 4: Modification of the BA11 linker

Two putative trypsin cleavage sites could be detected at the lysine and at the arginine position in the yol linker (KLEEGEFSEARV, SEQ ID NO: 129). In order to improve the stability of scFv BA11, the linker was modified: The lysin (K) was exchanged against isoleucin (I), arginin (R) against glycine (G) and the valin (V) against cystein (C). The resulting anti F4 scFv carries the designation myBA11 (BA11 with modified yol linker). The nucleotide and the amino acid sequence of myBA11 are depicted in SEQ ID NOs: 134 and 135.

In a second example the yol linker was replaced by a GS-linker (GGGGS)₂. The resulting antiF4 scFv carries the designation gsBA11. The nucleotide and the amino acid sequence of gsBA11 are depicted in SEQ ID NOs: 136 and 137. In a third approach two BA11 scFv fragments were coupled by a "double" GS-linker to form a bivalent BA11 (bvBA11). The amino acid sequence of bvBA11 is depicted in SEQ ID NO: 138. For gsBA11 and myBA11 similar expression rates were obtained in *E.coli* (up to 500µg/l).

### Example 5: Stability studies

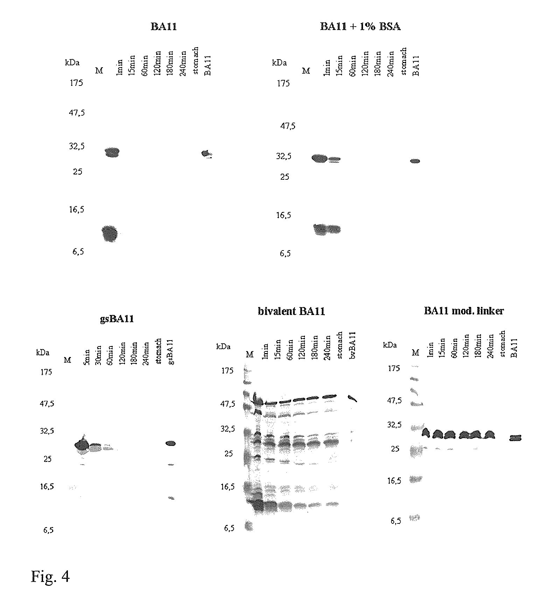

In order to examine the stability of the antibody fragments in the gastro-intestinal (GI) tract from pigs the antibody fragments were incubated with contents from stomach and small intestine. After incubation samples were analyzed by Western Blot to quantify the remaining antibody fragments. Best results were obtained with bvBA11 and myBA11 where the antibody fragment was stable for up to 240 min (fig. 4). In further experiments milk powder and/or BSA (1%) were added to the samples. In all cases this led to a stabilization of the antibody fragments in the GI probes.

### Example 6: Encapsulation test

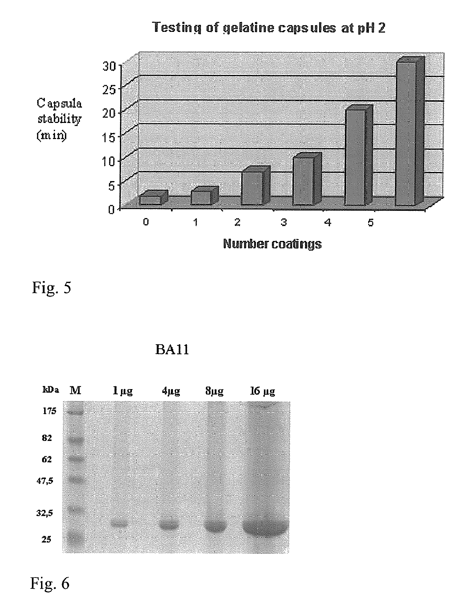

In order to delay the degradation of scFv BA11 in stomach material from feasted animals, acid-resistant capsules were used to achieve a delayed release of the antibody fragments. Conventional gelatin capsules were encased with a special liquid (cellulose acetate phthalate, CAP). The more often the capsules were coated with this liquid, the longer the antibody fragment was protected from low pH values (fig. 5).

### Example 7: Feeding study with antibody fragments against K88 (F4) fimbriae in piglets

Post-weaning piglets were infected with a high dose of an *E. coli* strain bearing F4 fimbriae. The treatment group (five animals) received scFv BA11 with the feed. The first dose was applied ca. 12 hrs before infection, thereafter the animals received a dose of 10mg/kgBW/day, distributed over three daily feed applications, for five days. Animals in the control group (seven animals) were infected without antibody fragment administration. All animals were sacrificed on day 4 and on day 5 respectively.

Infection with a highly concentrated *E. coli* broth was successful in all animals as shown by microbial examination of the feces. Clinical symptoms were observed only in one animal of the antibody group and in four animals of the control group.

All sick animals in the control group showed symptoms either immediately after infection or one day after whereas the one sick animal in the treatment group showed effects only late, i.e. on day 3. This animal was the only one with reduced body temperature, suggesting an increased sensitivity over all other animals included in this study.

Macroscopic analysis of the feces showed a more consistent quality in the treatment group (1/5 animals with lesser quality) compared to the control (6/7 animals with lesser quality). This was confirmed by determination of the dry weight which also showed marked higher values in the treatment group.

These data demonstrate that the single-chain antibody, BA11, has a protective and also a curative effect on *E.coli* infections. The number of infected animals was lower in the treatment group as was the pre-symptomatic effect as demonstrated by the analysis of fecal matter.

Another important aspect is the reduction of *E.coli* in the feces. While this is not directly correlated to the clinical aspects, it is of high importance for the spread of an infection in a population. A lower microbial burden means a lower infectious pressure, i.e. the risk for infections of healthy animals in a stable by an infected one is markedly reduced by application of the antibody fragment.

Samples from feces, urine, blood, muscle and intestine lymph nodes were taken and subjected to ELISA assays. The antibody fragment could not be detected in any of these samples.

In conclusion this infection trial with post-weaning piglets strongly indicates a preventative and also a curative effect of the orally applied single-chain antibody, BA11. Besides its direct effect on both clinical and pre-symptomatic symptoms, BA11 lessened the *E.coli* burden in the feces of infected animals and was thus able to reduce the infectious load in a population. Moreover it is safe as no scFv could be detected outside the GI tract, either in the animal itself nor in the feces.

### Example 8: Transient antibody fragment expression in plants

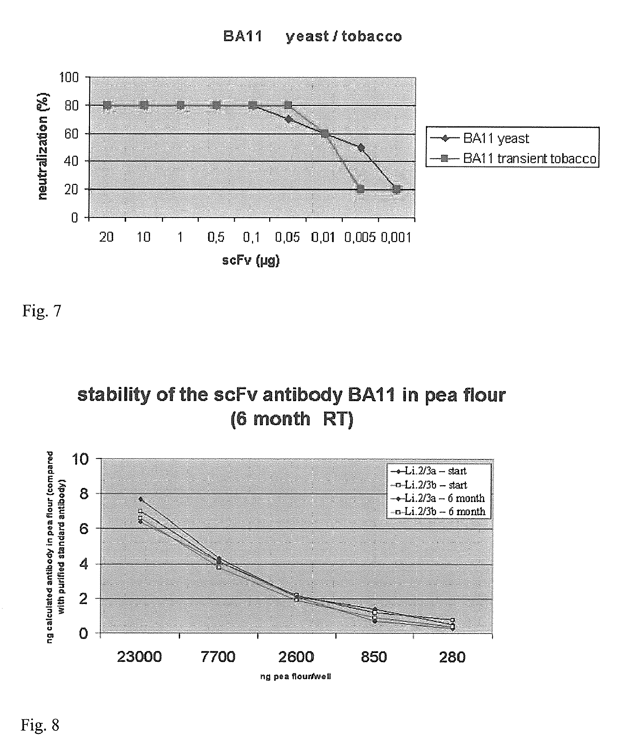

About 6 % scFv BA11 was purified from *Nicotiana benthamiana* from the total soluble protein after infection with Agrobacterium (MagnIcon®, IconGenetics GmbH, Halle, Germany). For protein extraction whole plants were used. Fig. 6 shows a freeze dried BA11 protein separated on a 12% SDS gel. After extraction the antibody fragment was tested by the brush border adhesion test. The results were similar to those obtained with BA11 expressed in yeast (fig. 7).

### Example 9: Stable plant transformation

The gene for scFv BA11 was transformed into tobacco under control of either the short or the long USB promoter. 89 lines were produced. BA11 was detected in 68 % of the 39 lines transformed with the short USB promoter. Using the long USB promoter only 10% were in the range of the detection limit. Five independent lines with the long and the short USB promoter are available which are being further characterized and propagated.

### Example 10: Stability tests of the pea line R46

Pea seeds containing the scFv antibody BA11 were milled and the meal stored for 6 months at room temperature. Antibody fragment activity (binding to the F4 antigen) was determined by ELISA. Fig. 8 represents stability analysis of the pea line R46 after 6 months.

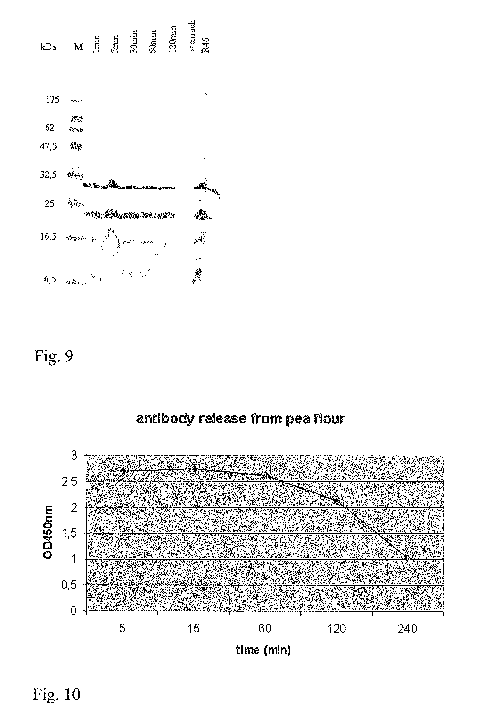

25mg pea powder of the line R46-18 (BA11 with long USB promoter) were incubated in 250/µl stomach contents of pigs. The antibody fragment concentration in the pea was about 2µg BA11/mg pea. scFv BA11 was not degraded in the stomach fluid for up to 120min as shown by Western blot (fig. 9). Thus pea offers a distinct protection for the antibody fragment in stomach fluid.

50mg flour of pea line R46-9/2 (BA11 with long USB promoter) were incubated with 100µl PBS buffer. At different time points the mixture was centrifuged and the supernatant was analyzed by ELISA. The pellet was then incubated again with 100µl PBS. The procedure was repeated up to 240min. During incubation with the PBS buffer the antibody fragment was slowly released from the pea flour (fig. 10).

### DESCRIPTION OF THE FIGURES

- Fig. 1:: Purified *E.coli* F4-fimbriae on a 15% SDS gel.
- Fig. 2A:: 12% SDS gel showing a dilution series of scFv-BA11 isolated from *Pichia pastoris.*
- Fig. 2B:: BA11 expressed in *E.coli* (12% SDS gel).
- Fig. 3:: Brush border adhesion test. With scFv = neutralization; the fimbriae of the bacteria are not able to attach to the brush borders (left). Without scFv = Fimbriae-bearing *E.coli* attach to the brush borders (right).
- Fig. 4:: Stability of some BA11-variants in small intestine content.
- Fig. 5:: Acid stability of gelatin capsules with increasing number of coatings.
- Fig. 6:: Freeze dried scFv BA11 purified from *Nicotiana benthamiana* and separated on a 12% SDS gel.
- Fig. 7:: Comparison of the inhibitory activity of scFv BA11 expressed in yeast and scFv BA11 transiently expressed in tobacco.
- Fig. 8:: Stability analysis of the pea line R46 after 6 months.
- Fig. 9:: Stability of the pea line R46 after 1 to 120min in stomach fluid.
- Fig. 10:: Release of the scFv antibody BA11 from pea flour in PBS at different time points.

## Claims

1. An antigen-binding polypeptide which specifically binds to F4 (K88)-fimbriae.

2. The antigen-binding polypeptide of claim 1, which specifically binds to a polypeptide comprising the amino acid sequence of SEQ ID NOs: 1, 2 and/or 3.

3. An antigen-binding polypeptide of claims 1 or 2, comprising:
a first polypeptide comprising the antigen-binding portion of the variable region of an antibody heavy chain, and
a second polypeptide comprising the antigen-binding portion of the variable region of an antibody light chain.

4. The antigen-binding polypeptide of claim 3, which further comprises a peptide linker linking said first and said second polypeptides into a single-chain polypeptide.

5. An antigen-binding polypeptide of claims 3 or 4, wherein said variable region of an antibody heavy chain comprises a VH CDR1, wherein the VH CDR1 has the amino acid sequence depicted in SEQ ID NO: 21.

6. The antigen-binding polypeptide of claim 5, wherein the VH CDR1 has the amino acid sequence depicted in SEQ ID NOs: 18, 19 or 20.

7. An antigen-binding polypeptide of any one of claims 3 to 6, wherein said variable region of an antibody heavy chain comprises a a VH CDR2, wherein the VH CDR2 has the amino acid sequence depicted in SEQ ID NO: 30.

8. The antigen-binding polypeptide of claim 7, wherein the VH CDR2 has the amino acid sequence depicted in SEQ ID NOs: 27, 31 or 33.

9. An antigen-binding polypeptide of any one of claims 3 to 8, wherein said variable region of an antibody heavy chain comprises a VH CDR3, wherein the VH CDR3 has the amino acid sequence depicted in SEQ ID NO: 40.

10. The antigen-binding polypeptide of claim 9, wherein the VH CDR3 has the amino acid sequence depicted in SEQ ID NOs: 37, 41 or 43.

11. An antigen-binding polypeptide of any one of claims 3 to 10, wherein said variable region of an antibody light chain comprises a VL CDR1, wherein the VL CDR1 has the amino acid sequence depicted in SEQ ID NO: 50.

12. The antigen-binding polypeptide of claim 11, wherein the VL CDR1 has the amino acid sequence depicted in SEQ ID NOs: 48, 49 or 51.

13. An antigen-binding polypeptide of any one of claims 3 to 12, wherein said variable region of an antibody light chain comprises a VL CDR2, wherein the VL CDR2 has the amino acid sequence depicted in SEQ ID NO: 60.

14. The antigen-binding polypeptide of claim 13, wherein the VL CDR2 has the amino acid sequence depicted in SEQ ID NOs: 58, 59 or 61.

15. An antigen-binding polypeptide of any one of claims 3 to 14, wherein said variable region of an antibody light chain comprises a VL CDR3, wherein the VL CDR3 has the amino acid sequence depicted in SEQ ID NO: 69.

16. The antigen-binding polypeptide of claim 15, wherein the VL CDR3 has the amino acid sequence depicted in SEQ ID NOs: 67, 68 or 70.

17. The single-chain antigen-binding polypeptide of claim 4 comprising the amino acid sequence of SEQ ID NOs: 4, 5, 6, 7, 8, 9 or 10.

18. An antigen-binding polypeptide of any one of claims 4 to 16, as far as they relate to a single-chain polypeptide, wherein said peptide linker is not susceptible to protease cleavage.

19. An antigen-binding polypeptide of any one of claims 4 to 16, as far as they relate to a single-chain polypeptide, wherein said peptide linker has an amino acid sequence of SEQ ID NO: 131.

20. An antigen-binding polypeptide of any one of claims 4 to 16, as far as they relate to a single-chain polypeptide, wherein said peptide linker is selected from the group consisting of a GS-linker, a yol-linker or a linker with the amino acid sequence of SEQ ID NO: 130.

21. A multivalent single-chain antigen-binding polypeptide, comprising two or more single-chain polypeptides of any one of claims 4 to 20, wherein the single-chain polypeptides are linked by a peptide linker.

22. The multivalent single-chain antigen-binding polypeptide of claim 20, wherein the peptide linker is a GS-linker.

23. An antigen-binding polypeptide of any one of claims 1 to 3 or 5 to 16, which is a Fab antibody fragment.

24. An antigen-binding polypeptide of claims 1 or 2, which is a non-immunoglobulin polypeptide.

25. A polynucleotide comprising a nucleotide sequence which encodes an antigen-binding polypeptide of any one of the preceding claims.

26. The polynucleotide of claim 25 comprising the nucleotide sequence of SEQ ID NOs: 11, 12, 13, 14, 15, 16 or 17.

27. A vector comprising the polynucleotide of claims 25 or 26.

28. The vector of claim 27 which is a phage display vector, a bacterial expression vector, a yeast expression vector, a fungus expression vector, an algae expression vector or a plant expression vector.

29. The plant expression vector of claim 28 comprising a seed specific promoter.

30. The plant expression vector of claim 29 wherein the promoter is the short or the long USB promoter.

31. A host cell comprising a polynucleotide of claim 25 or 26 or a vector of any one of claims 27 to 30.

32. The host cell of claim 31, which is a bacterial cell, a yeast cell, a fungus cell, an algae cell or a plant cell.

33. The host cell of claim 32, which is an *Escherichia coli* cell, a Streptomyces cell, a *Pichia pastoris* cell, a Schizosaccharomyces cell, an Aspergillus cell, a Chlamydomonas cell, *a Nicotiana spec.* cell, a *Pisum sativum* cell, a *Hordeum vulgare* cell, a *Zea mays* cell, a rapeseed cell, a *Cucumis sativus* cell or a *Linum usitatissimum* cell.

34. Transgenic plants or parts thereof or microorganisms containing a polynucleotide of claim 25 or 26 or a vector of any one of claims 27 to 30.

35. An antigen-binding polypeptide of any one of claims 1 to 24, which neutralizes the attachment of F4 (K88)-fimbriae to enterocytes.

36. An antigen-binding polypeptide of any one of claims 1 to 24, which has a protective and/or a curative effect on infection of mammals with bacteria expressing F4 (K88)-fimbriae.

37. A composition comprising the antigen-binding polypeptide of any one of claims 1 to 24, 35 and 36 and an acceptable excipient, carrier, buffer and/or stabilizer.

38. The composition of claim 37, wherein the excipient, carrier, buffer and/or stabilizer comprises a protein source, preferably BSA, milk powder and/or pea flour; micro-particles and/or gelatine capsules, which are preferably encased with a stabilizing liquid.

39. Use of an antigen-binding polypeptide of any one of claims 1 to 24, 35 and 36 for the manufacture of a medicament for the treatment and/or prevention of infections with bacteria expressing F4 (K88)-fimbriae.

40. Use of an antigen-binding polypeptide of any one of claims 1 to 24, 35 and 36 for detecting bacterial F4 (K88) fimbriae.

41. Use of the plants, plant parts or microorganisms of claim 32 as fodder for mammals.

42. Use according to claim 41, wherein the mammals are pigs.

43. A method of producing an antigen-binding polypeptide of any one of claims 1 to 24, 35 and 36, comprising:
providing an expression vector of any one of claims 27 to 30, and
expressing said vector in a host cell.


SEQUENCE LISTING

<110>  Novoplant GmbH

<120>  Antigen binding polypeptides against F4 (K88) fimbriae

<130>  N 7244

<160>  146

<170>  PatentIn version 3.3

<210>  1
<211>  285
<212>  PRT
<213>  Escherichia coli

<400>  1

Met Lys Lys Thr Leu Ile Ala Leu Ala Ile Ala Ala Ser Ala Ala Ser
1               5                   10                  15

Gly Met Ala His Ala Trp Met Thr Gly Asp Phe Asn Gly Ser Val Asp
            20                  25                  30

Ile Gly Gly Ser Ile Thr Ala Asp Asp Tyr Arg Gln Lys Trp Glu Trp
        35                  40                  45

Lys Val Gly Thr Gly Leu Asn Gly Phe Gly Asn Val Leu Asn Asp Leu
    50                  55                  60

Thr Asn Gly Gly Thr Lys Leu Thr Ile Thr Val Thr Gly Asn Lys Pro
65                  70                  75                  80

Ile Leu Leu Gly Arg Thr Lys Glu Ala Phe Ala Thr Pro Val Ser Gly
                85                  90                  95

Gly Val Asp Gly Ile Pro Gln Ile Ala Phe Thr Asp Tyr Glu Gly Ala
            100                 105                 110

Ser Val Lys Leu Arg Asn Thr Asp Gly Glu Thr Asn Lys Gly Leu Ala
        115                 120                 125

Tyr Phe Val Leu Pro Met Lys Asn Ala Glu Gly Thr Lys Val Gly Ser
    130                 135                 140

Val Lys Val Asn Ala Ser Tyr Ala Gly Val Phe Gly Lys Gly Gly Val
145                 150                 155                 160

Thr Ser Ala Asp Gly Glu Leu Phe Ser Leu Phe Ala Asp Gly Leu Arg
                165                 170                 175

Ala Ile Phe Tyr Gly Gly Leu Thr Thr Thr Val Ser Gly Ala Ala Leu
            180                 185                 190

Thr Ser Gly Ser Ala Ala Ala Ala Arg Thr Glu Leu Phe Gly Ser Leu

195                 200                 205

Ser Arg Asn Asp Ile Leu Gly Gln Ile Gln Arg Val Asn Ala Asn Ile
    210                 215                 220

Thr Ser Leu Val Asp Val Ala Gly Ser Tyr Arg Glu Asp Met Glu Tyr
225                 230                 235                 240

Thr Asp Gly Thr Val Val Ser Ala Ala Tyr Ala Leu Gly Ile Ala Asn
                245                 250                 255

Gly Gln Thr Ile Glu Ala Thr Phe Asn Gln Ala Val Thr Thr Ser Thr
            260                 265                 270

Gln Trp Ser Ala Pro Leu Asn Val Ala Ile Thr Tyr Tyr
        275                 280                 285

<210>  2
<211>  283
<212>  PRT
<213>  Escherichia coli

<400>  2
   Met Lys Lys Thr Leu Ile Ala Leu Ala Ile Ala Ala Ser Ala Ala Ser
   1               5                   10                  15
   Gly Met Ala His Ala Trp Met Thr Gly Asp Phe Asn Gly Ser Val Asp
               20                  25                  30
   Ile Gly Gly Ser Ile Thr Ala Asp Asp Tyr Arg Gln Lys Trp Glu Trp
           35                  40                  45
   Lys Val Gly Thr Gly Leu Asn Gly Phe Gly Asn Val Leu Asn Asp Leu
       50                  55                  60
   Thr Asn Gly Gly Thr Lys Leu Thr Ile Thr Val Thr Gly Asn Lys Pro
   65                  70                  75                  80
   Ile Leu Leu Gly Arg Thr Lys Glu Ala Phe Ala Thr Pro Val Thr Gly
                   85                  90                  95
   Gly Val Asp Gly Ile Pro His Ile Ala Phe Thr Asp Tyr Glu Gly Ala
               100                 105                 110
   Ser Val Val Leu Arg Asn Pro Asp Gly Glu Thr Asn Lys Lys Gly Leu
           115                 120                 125
   Ala Tyr Phe Val Leu Pro Met Lys Asn Ala Glu Gly Thr Lys Val Gly
       130                 135                 140
   Ser Val Lys Val Asn Ala Ser Tyr Ala Gly Val Leu Gly Arg Gly Gly
   145                 150                 155                 160
   Val Thr Ser Ala Asp Gly Glu Leu Leu Ser Leu Phe Ala Asp Gly Leu
                   165                 170                 175
   Ser Ser Ile Phe Tyr Gly Gly Leu Pro Arg Gly Ser Glu Leu Ser Ala
               180                 185                 190
   Gly Ser Ala Ala Ala Ala Arg Thr Lys Leu Phe Gly Ser Leu Ser Arg
           195                 200                 205
   Asn Asp Ile Leu Gly Gln Ile Gln Arg Val Asn Ala Asn Ile Thr Ser
       210                 215                 220
   Leu Val Asp Val Ala Gly Ser Tyr Arg Glu Asn Met Glu Tyr Thr Asp
   225                 230                 235                 240
   Gly Thr Val Val Ser Ala Ala Tyr Ala Leu Gly Ile Ala Asn Gly Gln
                   245                 250                 255
   Thr Ile Glu Ala Thr Phe Asn Gln Ala Val Thr Thr Ser Thr Gln Trp
               260                 265                 270
   Ser Ala Pro Leu Asn Val Ala Ile Thr Tyr Tyr
           275                 280

<210>  3
<211>  285
<212>  PRT
<213>  Escherichia coli

<400>  3

Met Lys Lys Thr Leu Ile Ala Leu Ala Ile Ala Ala Ser Ala Ala Ser
1               5                   10                  15

Gly Met Ala His Ala Trp Met Thr Gly Asp Phe Asn Gly Ser Val Asp
            20                  25                  30

Ile Gly Gly Ser Ile Thr Ala Asp Asp Tyr Arg Gln Lys Trp Glu Trp
        35                  40                  45

Lys Val Gly Thr Gly Leu Asn Gly Phe Gly Asn Val Leu Asn Asp Leu
    50                  55                  60

Thr Asn Gly Gly Thr Lys Leu Thr Ile Thr Val Thr Gly Asn Lys Pro
65                  70                  75                  80

Ile Leu Leu Gly Arg Thr Lys Glu Ala Phe Ala Thr Pro Val Thr Ser
                85                  90                  95

Gly Val Asp Gly Ile Pro His Ile Ala Phe Thr Asp Tyr Glu Gly Ala
            100                 105                 110

Ser Val Glu Leu Arg Asn Pro Asp Gly Glu Thr Glu Lys Gly Leu Ala
        115                 120                 125

Tyr Phe Val Leu Pro Met Lys Asn Ala Glu Gly Thr Lys Val Gly Ser
    130                 135                 140

Val Lys Val Asn Ala Ser Tyr Ala Gly Ala Leu Gly Arg Gly Gly Val
145                 150                 155                 160

Thr Ser Ala Asp Gly Glu Leu Met Ser Leu Phe Ala Glu Gly Ser His
                165                 170                 175

Ala Ile Phe Tyr Gly Gly Leu Pro Thr Asn Val Lys Asn Ser Glu Leu
            180                 185                 190

Lys Gly Gly Ser Ala Ala Ala Ala Arg Thr Glu Leu Phe Gly Ser Leu
        195                 200                 205

Ser Lys Asn Asp Ile Leu Gly Gln Ile Gln Arg Val Asn Ala Asn Ile
    210                 215                 220

Thr Ser Leu Val Asn Val Pro Gly Ser Phe Asn Glu Asn Met Ala Tyr
225                 230                 235                 240

Thr Asp Gly Ser Val Val Ser Val Ala Tyr Ala Leu Gly Ile Ala Asn
                245                 250                 255

Gly Gln Thr Ile Glu Ala Thr Phe Asn Gln Ala Val Thr Thr Ser Thr
            260                 265                 270

Gln Trp Ser Ala Pro Leu Asn Val Ala Ile Thr Tyr Tyr
        275                 280                 285

<210>  4
<211>  263
<212>  PRT
<213>  Artificial

<220>
<223>  BA61

<400>  4

Met Ala Glu Val Gln Leu Val Glu Ser Gly Gly Gly Leu Val Lys Pro
1               5                   10                  15

Gly Gly Ser Leu Lys Leu Ser Cys Ala Ala Ser Gly Phe Thr Phe Arg
            20                  25                  30

Asn Tyr Ala Met Ser Trp Val Arg Gln Ser Pro Glu Lys Arg Leu Glu
        35                  40                  45

Trp Val Ala Glu Ile Ser Thr Gly Gly Ser His Thr Tyr Tyr Pro Asp

50                  55                  60

Thr Val Thr Gly Arg Phe Thr Ile Ser Arg Asp Asn Ala Lys Asn Thr
65                  70                  75                  80

Leu Tyr Leu Glu Met Ser Ser Leu Arg Ser Glu Asp Thr Ala Met Tyr
                85                  90                  95

Tyr Cys Thr Arg Thr Gly Tyr Phe Asp Tyr Trp Gly Gln Gly Thr Thr
            100                 105                 110

Leu Thr Val Ser Ser Ala Lys Thr Thr Pro Pro Ser Val Lys Leu Glu
        115                 120                 125

Glu Gly Glu Phe Ser Glu Ala Arg Val Asp Ile Val Leu Thr Gln Ser
    130                 135                 140

Gln Lys Phe Met Ser Thr Ser Val Gly Asp Arg Val Ser Val Thr Cys
145                 150                 155                 160

Lys Ala Ser Gln Asn Val Gly Thr Asn Val Ala Trp Tyr Gln Gln Lys
                165                 170                 175

Pro Gly Gln Ser Pro Lys Ala Leu Ile Tyr Ser Ala Ser Tyr Arg Tyr
            180                 185                 190

Ser Gly Val Pro Asp Arg Phe Thr Gly Ser Gly Ser Gly Thr Asp Phe
        195                 200                 205

Thr Leu Thr Ile Ser Asn Val Gln Ser Glu Asp Leu Ala Glu Tyr Phe
    210                 215                 220

Cys Gln Gln Tyr Asn Ser Tyr Pro Tyr Thr Phe Gly Gly Gly Thr Lys
225                 230                 235                 240

Leu Glu Ile Lys Arg Ala Asp Ala Ala Pro Thr Val Ala Ala Ala Gly
                245                 250                 255

Ser His His His His His His
            260

<210>  5
<211>  263
<212>  PRT
<213>  Artificial

<220>
<223>  BA31

<400>  5

Met Ala Glu Val Gln Leu Val Glu Ser Gly Gly Gly Leu Val Lys Pro
1               5                   10                  15

Gly Gly Ser Leu Lys Leu Ser Cys Ala Ala Ser Gly Phe Thr Phe Arg
            20                  25                  30

Asn Tyr Ala Met Ser Trp Val Arg Gln Ser Pro Glu Lys Arg Leu Glu
        35                  40                  45

Trp Val Ala Glu Ile Ser Thr Ala Gly Ser Tyr Thr Tyr Tyr Pro Asp
    50                  55                  60

Thr Val Thr Gly Arg Phe Thr Ile Ser Arg Asp Asn Ala Lys Asn Thr
65                  70                  75                  80

Leu Tyr Leu Glu Met Ser Ser Leu Arg Ser Glu Asp Thr Ala Met Tyr
                85                  90                  95

Tyr Cys Thr Arg Thr Gly Tyr Phe Asp Tyr Trp Gly Gln Gly Thr Thr
            100                 105                 110

Leu Thr Val Ser Ser Ala Lys Thr Thr Pro Pro Ser Val Lys Leu Glu
        115                 120                 125

Glu Gly Glu Phe Ser Glu Ala Arg Val Asp Ile Gln Met Thr Gln Ser
    130                 135                 140

His Lys Phe Met Ser Thr Ser Val Gly Asp Arg Val Ser Ile Thr Cys
145                 150                 155                 160

Lys Ala Ser Gln Asp Val Gly Thr Thr Val Ala Trp Tyr Gln Gln Lys
                165                 170                 175

Pro Gly Gln Ser Pro Lys Leu Leu Ile Tyr Trp Ala Ser Thr Arg His
            180                 185                 190

Thr Gly Val Pro Asp Arg Phe Thr Gly Ser Gly Ser Gly Thr Asp Phe
        195                 200                 205

Thr Leu Thr Ile Ser Asn Val Gln Ser Glu Asp Leu Ala Asp Tyr Phe
    210                 215                 220

Cys Gln Gln Tyr Ser Ser Tyr Pro Tyr Thr Phe Gly Gly Gly Thr Lys
225                 230                 235                 240

Leu Glu Ile Lys Arg Ala Asp Ala Ala Pro Thr Val Ala Ala Ala Gly
                245                 250                 255

Ser His His His His His His
            260

<210>  6
<211>  263
<212>  PRT

<213>  Artificial

<220>
<223>  BA29

<400>  6

Met Ala Glu Val Gln Leu Val Glu Ser Gly Gly Gly Leu Val Lys Pro
1               5                   10                  15

Gly Gly Ser Leu Lys Leu Ser Cys Ala Ala Ser Gly Phe Pro Phe Arg
            20                  25                  30

Asn Tyr Ala Met Ser Trp Val Arg Gln Ser Pro Glu Lys Arg Leu Glu
        35                  40                  45

Trp Val Ala Glu Ile Ser Thr Ala Gly Ser Asn Thr Tyr Tyr Pro Asp
    50                  55                  60

Thr Val Thr Gly Arg Phe Thr Ile Ser Arg Asp Asn Ala Lys Asn Thr
65                  70                  75                  80

Leu Tyr Leu Glu Met Asn Ser Leu Arg Ser Glu Asp Thr Ala Met Tyr
                85                  90                  95

Tyr Cys Thr Arg Thr Gly Tyr Phe Asp Val Trp Gly Ala Gly Thr Ala
            100                 105                 110

Val Thr Val Ser Ser Ala Lys Thr Thr Pro Pro Ser Val Lys Leu Glu
        115                 120                 125

Glu Gly Glu Phe Ser Glu Ala Arg Val Asp Ile Val Met Thr Gln Ser
    130                 135                 140

Gln Lys Phe Met Ser Thr Ser Val Gly Asp Arg Val Ser Ile Thr Cys
145                 150                 155                 160

Lys Ala Ser Gln Asn Val Gly Thr Ala Val Ala Trp Tyr Gln Gln Lys
                165                 170                 175

Pro Gly Gln Ser Pro Lys Leu Leu Ile Tyr Ser Ala Ser Asn Arg Tyr
            180                 185                 190

Thr Gly Val Pro Asp Arg Phe Thr Gly Ser Gly Ser Gly Thr Asp Phe
        195                 200                 205

Thr Leu Thr Ile Ser Asn Met Gln Ser Glu Asp Leu Ala Asp Tyr Phe
    210                 215                 220

Cys Gln Gln Tyr Ser Ser Tyr Pro Tyr Thr Phe Gly Gly Gly Thr Lys
225                 230                 235                 240

Leu Glu Ile Lys Arg Ala Asp Ala Ala Pro Thr Val Ala Ala Ala Gly

245                 250                 255

Ser His His His His His His
            260

<210>  7
<211>  263
<212>  PRT
<213>  Artificial

<220>
<223>  BA3

<400>  7

Met Ala Glu Val Gln Leu Val Glu Ser Gly Gly Gly Leu Val Lys Pro
1               5                   10                  15

Gly Gly Ser Leu Lys Leu Ser Cys Ala Ala Ser Gly Phe Pro Phe Arg
            20                  25                  30

Asn Tyr Ala Met Ser Trp Val Arg Gln Ser Pro Glu Lys Arg Leu Glu
        35                  40                  45

Trp Val Ala Glu Ile Ser Thr Ala Gly Ser Asn Thr Tyr Tyr Pro Asp
    50                  55                  60

Thr Val Thr Gly Arg Phe Thr Ile Ser Arg Asp Asn Ala Lys Asn Thr
65                  70                  75                  80

Leu Tyr Leu Glu Met Asn Ser Leu Arg Ser Glu Asp Thr Ala Met Tyr
                85                  90                  95

Tyr Cys Ala Arg Thr Gly Tyr Phe Asp Val Trp Gly Ala Gly Thr Ala
            100                 105                 110

Val Thr Val Ser Ser Ala Lys Thr Thr Pro Pro Ser Val Lys Leu Glu
        115                 120                 125

Glu Gly Glu Phe Ser Glu Ala Arg Val Asp Ile Val Met Thr Gln Ser
    130                 135                 140

Gln Lys Phe Met Ser Thr Ser Val Gly Asp Arg Val Ser Val Thr Cys
145                 150                 155                 160

Lys Ala Ser Gln Asn Val Gly Thr Asn Val Ala Trp Tyr Gln Gln Lys
                165                 170                 175

Pro Gly Gln Ser Pro Lys Ala Leu Ile Tyr Ser Ala Ser Tyr Arg Tyr
            180                 185                 190

Ser Gly Val Pro Asp Arg Phe Thr Gly Ser Gly Ser Gly Thr Asp Phe
        195                 200                 205

Thr Leu Thr Ile Ser Asn Val Gln Ser Glu Asp Leu Ala Glu Tyr Phe
    210                 215                 220

Cys Gln Gln Tyr Asn Ser Tyr Pro Tyr Thr Phe Gly Gly Gly Thr Lys
225                 230                 235                 240

Leu Glu Ile Lys Arg Ala Asp Ala Ala Pro Thr Val Ala Ala Ala Gly
                245                 250                 255

Ser His His His His His His
            260

<210>  8
<211>  265
<212>  PRT
<213>  Artificial

<220>
<223>  BA11

<400>  8

Met Ala Glu Val Gln Leu Gln Gln Ser Gly Pro Glu Leu Val Lys Pro
1               5                   10                  15

Gly Ala Ser Val Lys Met Ser Cys Lys Ala Ser Gly Tyr Thr Phe Thr
            20                  25                  30

Asn Tyr Val Ile His Trp Val Lys Gln Lys Pro Gly Gln Gly Leu Glu
        35                  40                  45

Trp Ile Gly Tyr Ile Asn Pro Tyr Asn Asp Phe Thr Lys Tyr Asn Glu
    50                  55                  60

Lys Phe Lys Gly Lys Ala Thr Leu Thr Ser Asp Lys Ser Ser Ser Thr
65                  70                  75                  80

Ala Tyr Met Glu Leu Ser Ser Leu Thr Ser Glu Asp Ser Ala Val Tyr
                85                  90                  95

Tyr Cys Thr Arg Val Gly Glu Thr Pro Phe Ala Tyr Trp Gly Gln Gly
            100                 105                 110

Thr Leu Val Thr Val Ser Ala Ala Lys Thr Thr Pro Pro Ser Val Lys
        115                 120                 125

Leu Glu Glu Gly Glu Phe Ser Glu Ala Arg Val Asp Ile Gln Met Thr
    130                 135                 140

Gln Thr Thr Ser Ser Leu Ser Ala Ser Leu Gly Asp Arg Val Thr Ile
145                 150                 155                 160

Ser Cys Arg Ala Ser Gln Asp Ile Ser Asn Tyr Leu Asn Trp Tyr Gln

165                 170                 175

Gln Lys Pro Asp Gly Thr Val Lys Leu Leu Ile Tyr Tyr Thr Ser Arg
            180                 185                 190

Leu His Ser Gly Val Pro Ser Arg Phe Ser Gly Ser Gly Ser Gly Thr
        195                 200                 205

Asp Tyr Ser Leu Thr Ile Ser Asn Leu Glu Gln Glu Asp Ile Ala Thr
    210                 215                 220

Tyr Phe Cys Gln Gln Gly Asn Thr Leu Pro Trp Thr Phe Gly Gly Gly
225                 230                 235                 240

Thr Lys Leu Glu Ile Lys Arg Pro Asp Ala Ala Pro Thr Val Ala Ala
                245                 250                 255

Ala Gly Ser His His His His His His
            260                 265

<210>  9
<211>  264
<212>  PRT
<213>  Artificial

<220>
<223>  BA67

<400>  9

Met Ala Glu Val Gln Leu Gln Gln Ser Gly Pro Glu Leu Val Arg Pro
1               5                   10                  15

Gly Val Ser Val Lys Ile Ser Cys Lys Gly Ser Gly Tyr Thr Phe Thr
            20                  25                  30

Asp Tyr Ala Met His Trp Val Lys Gln Ser His Ala Lys Ser Leu Glu
        35                  40                  45

Trp Ile Gly Val Ile Ser Thr Tyr Ser Gly Ser Thr Asn Tyr Asn Gln
    50                  55                  60

Ser Phe Lys Gly Lys Ala Thr Met Thr Val Asp Lys Ser Ser Ser Thr
65                  70                  75                  80

Ala Tyr Met Glu Leu Ala Arg Leu Thr Ser Glu Asp Ser Ala Leu Tyr
                85                  90                  95

Tyr Cys Ala Arg Thr Asp Tyr Pro Tyr Phe Tyr Ala Met Asp Tyr Trp
            100                 105                 110

Gly Gln Gly Thr Ser Val Thr Val Ser Ser Ala Lys Thr Thr Pro Pro
        115                 120                 125

Ser Val Lys Leu Glu Glu Gly Glu Phe Ser Glu Ala Arg Val Gln Ser
    130                 135                 140

Val Leu Thr Gln Pro Pro Ser Val Ser Ala Ala Pro Gly Gln Lys Val
145                 150                 155                 160

Thr Ile Ser Cys Ser Gly Ser Ser Ser Asn Ile Gly Asn Asn Tyr Val
                165                 170                 175

Ser Trp Tyr Val Gln Leu Pro Gly Thr Ala Pro Lys Leu Leu Ile Tyr
            180                 185                 190

Asp Asn Asn Lys Arg Phe Ser Gly Val Pro Asp Arg Phe Ser Gly Ser
        195                 200                 205

Lys Ser Gly Thr Ser Ala Thr Leu Gly Ile Thr Gly Leu Gln Thr Gly
    210                 215                 220

Asp Glu Ala Asp Tyr Tyr Cys Gly Ala Trp Asp Gly Ser Leu Arg Glu
225                 230                 235                 240

Ala Val Phe Gly Gly Gly Thr Lys Val Thr Val Leu Gly Ala Ala Ala
                245                 250                 255

Gly Ser His His His His His His
            260

<210>  10
<211>  268
<212>  PRT
<213>  Artificial

<220>
<223>  BA5

<400>  10

Met Ala Glu Val Gln Leu Gln Gln Ser Gly Ala Glu Leu Ala Arg Pro
1               5                   10                  15

Gly Ala Ser Val Lys Ile Ser Cys Lys Gly Ser Gly Tyr Thr Phe Thr
            20                  25                  30

Asp Tyr Ala Met His Trp Val Lys Gln Ser His Ala Lys Ser Leu Glu
        35                  40                  45

Trp Ile Gly Val Ile Ser Thr Tyr Ser Gly Ser Thr Asn Tyr Asn Gln
    50                  55                  60

Lys Phe Lys Asp Lys Ala Thr Met Thr Val Asp Lys Ser Thr Ser Thr
65                  70                  75                  80

Ala Tyr Val Glu Leu Ala Arg Leu Thr Ser Glu Asp Thr Ala Ile Tyr

                85                  90                  95

Tyr Cys Ala Arg Thr Asn Tyr Pro Tyr Phe Asn Gly Met Asp Tyr Trp
            100                 105                 110

Gly Gln Gly Thr Ser Val Thr Val Ser Ser Ala Lys Thr Thr Pro Pro
        115                 120                 125

Ser Val Lys Leu Glu Glu Gly Glu Phe Ser Glu Ala Arg Val Asp Val
    130                 135                 140

Val Met Thr Gln Thr Gln Lys Phe Met Ser Thr Ser Val Gly Asp Arg
145                 150                 155                 160

Val Ser Val Thr Cys Lys Ala Ser Gln Asn Val Gly Thr Asn Val Ala
                165                 170                 175

Trp Tyr Gln Gln Lys Pro Gly Gln Ser Pro Lys Ala Leu Ile Tyr Ser
            180                 185                 190

Ala Ser Tyr Arg Tyr Ser Gly Val Pro Asp Arg Phe Thr Gly Ser Gly
        195                 200                 205

Ser Gly Thr Asp Phe Thr Leu Thr Ile Ser Asn Val Gln Ser Glu Asp
    210                 215                 220

Leu Ala Glu Tyr Phe Cys Gln Gln Tyr Asn Ser Tyr Pro Tyr Thr Phe
225                 230                 235                 240

Gly Gly Gly Thr Lys Leu Glu Ile Lys Arg Ala Asp Ala Ala Pro Thr
                245                 250                 255

Val Ala Ala Ala Gly Ser His His His His His His
            260                 265

<210> 11
<211> 789
<212> DNA
<213> Artificial

<220>
<223> BA61

<400> 11
atggcggaag tgcagctggt ggagtctggg ggaggcttag tgaagcctgg agggtccctg 60

aaactctcct gtgcagcctc tggattcact ttcaggaact atgccatgtc ttgggttcgc 120

cagtctccag agaagagact ggagtgggtc gcagaaatta gtactggtgg tagtcacacc 180

tactatccag acactgtgac gggccgattc accatctcca gagacaatgc caagaacacc 240

ctatacctgg aaatgagcag tctgaggtct gaggacacgg ccatgtatta ctgtacaaga 300

actggttact ttgactactg gggccagggc accactctca cagtctcctc agccaaaaca 360

acacccccat cagtcaagct tgaagaaggt gaattttcag aagcacgcgt agacattgtg 420

ctgacacagt cccaaaaatt catgtccaca tcagtaggag acagggtcag cgtcacctgc 480

aaggccagtc agaatgtggg tactaatgta gcctggtatc aacagaaacc agggcaatct 540

cctaaagcac tgatttactc ggcatcctac cggtacagtg gagtccctga tcgcttcaca 600

ggcagtggat ctgggacaga tttcactctc accatcagca atgtgcagtc tgaagacttg 660

gcagagtatt tctgtcagca atataacagc tatccttaca cgttcggagg ggggaccaag 720

ctggaaataa aacgggctga tgctgcacca actgtagcgg ccgctggatc ccatcaccat 780

caccatcac 789

<210> 12
<211> 789
<212> DNA
<213> Artificial

<220>
<223> BA31

<400> 12
atggcggaag tgcagctggt ggagtctggg ggaggcttag tgaagcctgg agggtccctg 60

aaactctcct gtgcagcctc tggattcact ttcagaaact atgccatgtc ttgggttcgc 120

cagtctccag agaagaggct ggagtgggtc gcagaaatta gtactgctgg tagttacacc 180

tactatccag acactgtgac gggccgattc accatctcca gagacaatgc caagaacacc 240

ctgtacctgg aaatgagcag tctgaggtct gaggacacgg ccatgtatta ctgtacaaga 300

actggttact ttgactactg gggccaaggc accactctca cagtctcctc agccaaaaca 360

acacccccat cagtcaagct tgaagaaggt gaattttcag aagcacgcgt agacatccag 420

atgacacagt ctcacaaatt catgtccaca tcagtaggag acagggtcag catcacctgc 480

aaggccagtc aggatgtggg tactactgta gcctggtatc aacagaaacc agggcaatct 540

cctaaactac tgatttactg ggcatccacc cggcacactg gagtccctga tcgcttcaca 600

ggcagtggat ctgggacaga tttcactctc accattagca atgtgcagtc tgaagacttg 660

gcagattatt tctgtcagca atatagcagc tatccgtaca cgttcggagg ggggaccaag 720

ctggaaatta aacgggctga tgctgcacca actgtagcgg ccgctggatc ccatcaccat 780

caccatcac 789

<210> 13
<211> 789
<212> DNA
<213> Artificial

<220>
<223> BA29

<400> 13
atggcggaag tgcagctggt ggagtctggg ggaggcttag tgaagcctgg agggtccctg 60

aaactctcct gtgcagcctc tggattccct ttcagaaact atgccatgtc ctgggttcgc 120

cagtccccag agaagaggct ggagtgggtc gcagaaatta gtactgctgg tagtaacacc 180

tactatccag acactgtgac gggccgattc accatctcca gagacaatgc caagaacacc 240

ctgtacctgg aaatgaacag tctgaggtct gaggacacgg ccatgtatta ctgtacaagg 300

acggggtact tcgatgtctg gggcgcaggg actgcggtca ccgtctcctc agccaaaaca 360

acacccccat cagtcaagct tgaagaaggt gaattttcag aagcacgcgt agacattgtg 420

atgactcagt ctcaaaaatt catgtccaca tcagtaggag acagggtcag catcacctgc 480

aaggccagtc agaatgtggg tactgctgta gcctggtatc aacagaaacc aggacaatct 540

cctaaactac tgatttactc ggcatccaat cggtacactg gagtccctga tcgcttcaca 600

ggcagtggat ctgggacaga tttcactctc accatcagca atatgcagtc tgaagacctg 660

gcagattatt tctgccagca atatagcagc tatccgtaca cgttcggagg ggggaccaag 720

ctggaaataa aacgggctga tgctgcacca actgtagcgg ccgctggatc ccatcaccat 780

caccatcac 789

<210> 14
<211> 789
<212> DNA
<213> Artificial

<220>
<223> BA3

<400> 14
atggcggaag tgcagctggt ggagtctggg ggaggcttag tgaagcctgg agggtccctg 60

aaactctcct gtgcagcctc tggattccct ttcagaaact atgccatgtc ctgggttcgc 120

cagtccccag agaagaggct ggagtgggtc gcagaaatta gtactgctgg tagtaacacc 180

tactatccag acactgtgac gggccgattc accatctcca gagacaatgc caagaacacc 240

ctgtacctgg aaatgaacag tctgaggtct gaggacacgg ccatgtatta ctgtgcaagg 300

acggggtact tcgatgtctg gggcgcaggg actgcggtca ccgtctcctc agccaaaaca 360

acacccccat cagtcaagct tgaagaaggt gaattttcag aagcacgcgt agacattgtg 420

atgacccagt ctcaaaaatt catgtccaca tcagtaggag acagggtcag cgtcacctgc 480

aaggccagtc agaatgtggg tactaatgta gcctggtatc aacagaaacc agggcaatct 540

cctaaagcac tgatttactc ggcatcctac cggtacagtg gagtccctga tcgcttcaca 600

ggcagtggat ctgggacaga tttcactctc accatcagca atgtgcagtc tgaagacttg 660

gcagagtatt tctgtcagca atataacagc tatccttaca cgttcggagg ggggaccaag 720

ctggaaataa aacgggctga tgctgcacca actgtagcgg ccgctggatc ccatcaccat 780

caccatcac 789

<210> 15
<211> 795
<212> DNA
<213> Artificial

<220>
<223>  BA11

<400>  15
atggcggagg tccagctgca gcagtctgga cctgagctgg taaagcctgg ggcttcagtg      60

aagatgtcct gcaaggcttc tggatacaca ttcactaact atgttataca ctgggtgaag     120

cagaagcctg ggcagggcct tgagtggatt ggatatatta atccttacaa tgattttact     180

aaatacaatg agaagttcaa aggcaaggcc acactgactt cagacaaatc ctccagcaca     240

gcctacatgg agctcagcag cctgacctct gaggactctg cggtctatta ctgtacaaga     300

gtcggggaaa ccccctttgc ttactggggc caagggactc tggtcactgt ctctgcagcc     360

aaaacaacac ccccatcagt caagcttgaa gaaggtgaat tttcagaagc acgcgtagat     420

atccagatga cacagactac atcctccctg tctgcctctc tgggagacag agtcaccatc     480

agttgcaggg caagtcagga cattagcaat tatttaaact ggtatcagca gaaaccagat     540

ggaactgtta aactcctgat ctactacaca tcaagattac actcaggagt cccatcaagg     600

ttcagtggca gtgggtctgg aacagattat tctctcacca ttagcaacct ggagcaagaa     660

gatattgcca cttacttttg ccaacagggt aatacgcttc cgtggacgtt cggtggaggc     720

accaagctgg aaatcaaacg gcctgatgct gcaccaactg tagcggccgc tggatcccat     780

caccatcacc atcac                                                      795

<210>  16
<211>  792
<212>  DNA
<213>  Artificial

<220>
<223>  BA67

<400>  16
atggcggagg tccagctgca acaatctggg cctgagctgg tgaggcctgg agtctcagtg      60

aagatttcct gcaagggttc cggctacaca ttcactgatt atgctatgca ctgggtgaag     120

cagagtcatg caaagagtct agagtggatt ggagtgatta gtacttactc tggtagtaca     180

aactacaacc agagctttaa gggcaaggcc acaatgactg tagacaaatc ctccagcaca     240

gcctatatgg aacttgccag attgacatct gaggattctg ccctctatta ctgtgcaaga     300

acggactacc cttatttcta tgctatggac tactggggtc agggaacctc agtcaccgtc     360

tcctcagcca aaacaacacc cccatcagtc aagcttgaag aaggtgaatt ttcagaagca     420

cgcgtacagt ctgtgttgac tcagccgccc tcagtgtctg cggccccagg acagaaggtc     480

accatctcct gctctggaag cagctccaac attggaaata attatgtctc ctggtacgtt     540

caactcccag gaacagcccc caaactcctc atttatgaca ataataagcg attctcagga     600

gttcctgacc gattctctgg ctccaagtct ggcacgtcag ccaccctggg catcaccggg     660

ctccagactg gggacgaggc cgattattac tgcggagcat gggatggcag cctgcgtgaa     720

gcggtattcg gcggagggac caaggtcacc gtcctaggtg cggccgctgg atcccatcac     780

catcaccatc ac                                                          792

<210>  17
<211>  804
<212>  DNA
<213>  Artificial

<220>
<223>  BA5

<400>  17
atggcggagg ttcagctcca gcagtctggg gctgaactgg caagacctgg ggcctcagtg      60

aagatttcct gcaagggttc cggctacaca ttcactgatt atgctatgca ctgggtgaag     120

cagagtcatg caaagagtct agagtggatt ggagttatta gtacttactc tggtagtaca     180

aactacaacc agaagtttaa ggacaaggcc acaatgactg tagacaaatc caccagcaca     240

gcctatgtgg aacttgccag attgacatct gaggatactg ccatctatta ctgtgcaaga     300

acaaactacc cttatttcaa tggtatggac tactggggtc aaggaacctc agtcaccgtc     360

tcctcagcca aaacaacacc cccatcagtc aagcttgaag aaggtgaatt ttcagaagca     420

cgcgtagatg ttgtgatgac ccagactcaa aaattcatgt ccacatcagt aggagacagg     480

gtcagcgtca cctgcaaggc cagtcagaat gtgggtacta atgtagcctg gtatcaacag     540

aaaccagggc aatctcctaa agcactgatt tactcggcat cctaccggta cagtggagtc     600

cctgatcgct tcacaggcag tggatctggg acagatttca ctctcaccat cagcaatgtg     660

cagtctgaag acttggcaga gtatttctgt cagcaatata acagctatcc ttacacgttc     720

ggagggggga ccaagctgga aataaaacgg gctgatgctg caccaactgt agcggccgct     780

ggatcccatc accatcacca tcac                                            804

<210>  18
<211>  5
<212>  PRT
<213>  Artificial

<220>
<223>  VH CDR1 of BA61 + 31 + 29 + 3

<400>  18

Asn Tyr Ala Met Ser
1               5

<210>  19
<211>  5
<212>  PRT
<213>  Artificial

<220>
<223>  VH CDR1 of BA11

<400>  19

Asn Tyr Val Ile His
1               5

<210>  20
<211>  5
<212>  DNA
<213>  Artificial

<220>
<223>  VH CDR1 of BA67 + 5

<400>  20
dyamh                                                                    5


<210>  21
<211>  5
<212>  PRT
<213>  Artificial

<220>
<223>  consensus amino acid sequence of VH CDR1 of all polypeptides


<220>
<221>  misc_feature
<222>  (1)..(1)
<223>  Xaa can be any naturally occurring amino acid

<220>
<221>  misc_feature
<222>  (3)..(5)
<223>  Xaa can be any naturally occurring amino acid

<400>  21

Xaa Tyr Xaa Xaa Xaa
1               5


<210>  22
<211>  5
<212>  PRT
<213>  Artificial

<220>
<223>  consensus amino acid sequence of VH CDR1 of BA11 + 67 + 5


<220>
<221>  misc_feature
<222>  (1)..(1)
<223>  Xaa can be any naturally occurring amino acid

<220>
<221>  misc_feature
<222>  (3)..(4)
<223>  Xaa can be any naturally occurring amino acid

<400>  22

Xaa Tyr Xaa Xaa His
1               5


<210>  23
<211>  5
<212>  PRT
<213>  Artificial

<220>
<223>  consensus amino acid sequence of VH CDR1 of BA61+31+29+3+11


<220>
<221>  misc_feature
<222>  (3)..(5)
<223>  Xaa can be any naturally occurring amino acid

<400>  23

Asn Tyr Xaa Xaa Xaa
1               5


<210>  24
<211>  17
<212>  PRT
<213>  Artificial

<220>
<223>  VH CDR2 of BA61

<400>  24

Glu Ile Ser Thr Gly Gly Ser His Thr Tyr Tyr Pro Asp Thr Val Thr
1               5                   10                  15


Gly



<210>  25
<211>  17
<212>  PRT
<213>  Artificial

<220>
<223>  VH CDR2 of BA31

<400>  25

Glu Ile Ser Thr Ala Gly Ser Tyr Thr Tyr Tyr Pro Asp Thr Val Thr
1               5                   10                  15


Gly



<210>  26
<211>  17
<212>  PRT
<213>  Artificial

<220>
<223>  VH CDR2 of BA29 + 3

<400>  26

Glu Ile Ser Thr Ala Gly Ser Asn Thr Tyr Tyr Pro Asp Thr Val Thr
1               5                   10                  15


Gly

<210>  27
<211>  17
<212>  PRT
<213>  Artificial

<220>
<223>  VH CDR2 of BA11

<400>  27

Tyr Ile Asn Pro Tyr Asn Asp Phe Thr Lys Tyr Asn Glu Lys Phe Lys
1               5                   10                  15


Gly



<210>  28
<211>  17
<212>  PRT
<213>  Artificial

<220>
<223>  VH CDR2 of BA67

<400>  28

Val Ile Ser Thr Tyr Ser Gly Ser Thr Asn Tyr Asn Gln Ser Phe Lys
1               5                   10                  15


Gly



<210>  29
<211>  17
<212>  PRT
<213>  Artificial

<220>
<223>  VH CDR2 of BA5

<400>  29

Val Ile Ser Thr Tyr Ser Gly Ser Thr Asn Tyr Asn Gln Lys Phe Lys
1               5                   10                  15


Asp



<210>  30
<211>  17
<212>  PRT
<213>  Artificial

<220>
<223>  consensus amino acid sequence of VH CDR2 of all polypeptides


<220>
<221>  misc_feature
<222>  (1)..(1)
<223>  Xaa can be any naturally occurring amino acid

<220>
<221>  misc_feature
<222>  (3)..(8)
<223>  Xaa can be any naturally occurring amino acid

<220>
<221>  misc_feature
<222>  (10)..(10)
<223>  Xaa can be any naturally occurring amino acid

<220>
<221>  misc_feature
<222>  (12)..(17)
<223>  Xaa can be any naturally occurring amino acid

<400>  30

Xaa Ile Xaa Xaa Xaa Xaa Xaa Xaa Thr Xaa Tyr Xaa Xaa Xaa Xaa Xaa
1               5                   10                  15


Xaa




<210>  31
<211>  17
<212>  PRT
<213>  Artificial

<220>
<223>  consensus amino acid sequence of VH CDR2 of BA61 + 31 + 29 + 3


<220>
<221>  misc_feature
<222>  (5)..(5)
<223>  Xaa can be any naturally occurring amino acid

<220>
<221>  misc_feature
<222>  (8)..(8)
<223>  Xaa can be any naturally occurring amino acid

<400>  31

Glu Ile Ser Thr Xaa Gly Ser Xaa Thr Tyr Tyr Pro Asp Thr Val Thr
1               5                   10                  15


Gly




<210>  32
<211>  17
<212>  PRT
<213>  Artificial

<220>
<223>  consensus amino acid sequence of VH CDR2 of BA 11 + 67 + 5


<220>
<221>  misc_feature
<222>  (1)..(1)
<223>  Xaa can be any naturally occurring amino acid

<220>
<221>  misc_feature
<222>  (3)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (10)..(10)
<223>  Xaa can be any naturally occurring amino acid

<220>
<221>  misc_feature
<222>  (13)..(14)
<223>  Xaa can be any naturally occurring amino acid

<220>
<221>  misc_feature
<222>  (17)..(17)
<223>  Xaa can be any naturally occurring amino acid

<400>  32

Xaa Ile Xaa Xaa Tyr Xaa Xaa Xaa Thr Xaa Tyr Asn Xaa Xaa Phe Lys
1               5                   10                  15


Xaa




<210>  33
<211>  17
<212>  PRT
<213>  Artificial

<220>
<223>  consensus amino acid sequence of VH CDR2 of BA 67 + 5


<220>
<221>  misc_feature
<222>  (14)..(14)
<223>  Xaa can be any naturally occurring amino acid

<220>
<221>  misc_feature
<222>  (17)..(17)
<223>  Xaa can be any naturally occurring amino acid

<400>  33

Val Ile Ser Thr Tyr Ser Gly Ser Thr Asn Tyr Asn Gln Xaa Phe Lys
1               5                   10                  15


Xaa




<210>  34
<211>  17
<212>  PRT

<213>  Artificial

<220>
<223>  consensus amino acid sequence of VH CDR2 of BA61 + 31+29+3+11


<220>
<221>  misc_feature
<222>  (1)..(1)
<223>  Xaa can be any naturally occurring amino acid

<220>
<221>  misc_feature
<222>  (3)..(8)
<223>  Xaa can be any naturally occurring amino acid

<220>
<221>  misc_feature
<222>  (10)..(10)
<223>  Xaa can be any naturally occurring amino acid

<220>
<221>  misc_feature
<222>  (12)..(16)
<223>  Xaa can be any naturally occurring amino acid

<400>  34

Xaa Ile Xaa Xaa Xaa Xaa Xaa Xaa Thr Xaa Tyr Xaa Xaa Xaa Xaa Xaa
1               5                   10                  15


Gly



<210>  35
<211>  6
<212>  PRT
<213>  Artificial

<220>
<223>  VH CDR3 of BA61 + 31

<400>  35

Thr Gly Tyr Phe Asp Tyr
1               5


<210>  36
<211>  6
<212>  PRT
<213>  Artificial

<220>
<223>  VH CDR3 of BA29 + 3

<400>  36

Thr Gly Tyr Phe Asp Val
1               5


<210>  37
<211>  8
<212>  PRT
<213>  Artificial

<220>
<223>  VH CDR3 of BA11

<400>  37

Val Gly Glu Thr Pro Phe Ala Tyr
1               5


<210>  38
<211>  11
<212>  PRT
<213>  Artificial

<220>
<223>  VH CDR3 of BA67

<400>  38

Thr Asp Tyr Pro Tyr Phe Tyr Ala Met Asp Tyr
1               5                   10


<210>  39
<211>  11
<212>  PRT
<213>  Artificial

<220>
<223>  VH CDR3 of BA5

<400>  39

Thr Asn Tyr Pro Tyr Phe Asn Gly Met Asp Tyr
1               5                   10


<210>  40
<211>  11
<212>  PRT
<213>  Artificial

<220>
<223>  consensus amino acid sequence of VH CDR3 of all polypeptides


<220>
<221>  misc_feature
<222>  (1)..(3)
<223>  Xaa can be any naturally occurring amino acid

<220>
<221>  misc_feature
<222>  (7)..(11)
<223>  Xaa can be any naturally occurring amino acid

<400>  40

Xaa Xaa Xaa Pro Tyr Phe Xaa Xaa Xaa Xaa Xaa
1               5                   10


<210>  41
<211>  6
<212>  PRT
<213>  Artificial

<220>
<223>  consensus amino acid sequence of VH CDR3 of BA 61 + 31 + 29 + 3


<220>
<221>  misc_feature
<222>  (6)..(6)
<223>  Xaa can be any naturally occurring amino acid

<400>  41

Thr Gly Tyr Phe Asp Xaa
1               5


<210>  42
<211>  11
<212>  PRT
<213>  Artificial

<220>
<223>  consensus amino acid sequence of VH CDR3 of BA 11 + 67 + 5


<220>
<221>  misc_feature
<222>  (1)..(3)
<223>  Xaa can be any naturally occurring amino acid

<220>
<221>  misc_feature
<222>  (7)..(10)
<223>  Xaa can be any naturally occurring amino acid

<400>  42

Xaa Xaa Xaa Pro Tyr Phe Xaa Xaa Xaa Xaa Tyr
1               5                   10


<210>  43
<211>  11
<212>  PRT
<213>  Artificial

<220>
<223>  consensus amino acid sequence of VH CDR3 of BA 67 + 5


<220>
<221>  misc_feature
<222>  (2)..(2)
<223>  Xaa can be any naturally occurring amino acid

<220>
<221>  misc_feature
<222>  (7)..(8)
<223>  Xaa can be any naturally occurring amino acid

<400>  43

Thr Xaa Tyr Pro Tyr Phe Xaa Xaa Met Asp Tyr
1               5                   10


<210>  44
<211>  8
<212>  PRT

<213>  Artificial

<220>
<223>  consensus amino acid sequence of VH CDR3 of BA61 + 31+29+3+11


<220>
<221>  misc_feature
<222>  (1)..(1)
<223>  Xaa can be any naturally occurring amino acid

<220>
<221>  misc_feature
<222>  (3)..(3)
<223>  Xaa can be any naturally occurring amino acid

<220>
<221>  misc_feature
<222>  (7)..(8)
<223>  Xaa can be any naturally occurring amino acid

<400>  44

Xaa Gly Xaa Thr Pro Phe Xaa Xaa
1               5


<210>  45
<211>  11
<212>  PRT
<213>  Artificial

<220>
<223>  VL CDR1 of BA61 + 3 + 5

<400>  45

Lys Ala Ser Gln Asn Val Gly Thr Asn Val Ala
1               5                   10


<210>  46
<211>  11
<212>  PRT
<213>  Artificial

<220>
<223>  VL CDR1 of BA29

<400>  46

Lys Ala Ser Gln Asn Val Gly Thr Ala Val Ala
1               5                   10


<210>  47
<211>  11
<212>  PRT
<213>  Artificial

<220>
<223>  VL CDR1 of BA31

<400>  47

Lys Ala Ser Gln Asp Val Gly Thr Thr Val Ala
1               5                   10

<210>  48
<211>  11
<212>  PRT
<213>  Artificial

<220>
<223>  VL CDR1 of BA11

<400>  48

Arg Ala Ser Gln Asp Ile Ser Asn Tyr Leu Asn
1               5                   10


<210>  49
<211>  13
<212>  PRT
<213>  Artificial

<220>
<223>  VL CDR1 of BA67

<400>  49

Ser Gly Ser Ser Ser Asn Ile Gly Asn Asn Tyr Val Ser
1               5                   10


<210>  50
<211>  13
<212>  PRT
<213>  Artificial

<220>
<223>  consensus amino acid sequence of VL CDR1 of all polypeptides


<220>
<221>  misc_feature
<222>  (1)..(2)
<223>  Xaa can be any naturally occurring amino acid

<220>
<221>  misc_feature
<222>  (4)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (10)..(13)
<223>  Xaa can be any naturally occurring amino acid

<400>  50

Xaa Xaa Ser Xaa Ser Xaa Xaa Xaa Asn Xaa Xaa Xaa Xaa
1               5                   10


<210>  51
<211>  11
<212>  PRT
<213>  Artificial

<220>
<223>  consensus amino acid sequence of VL CDR1 of BA61 + 3+5+29+31


<220>
<221>  misc_feature
<222>  (5)..(5)
<223>  Xaa can be any naturally occurring amino acid

<220>
<221>  misc_feature
<222>  (9)..(9)
<223>  Xaa can be any naturally occurring amino acid

<400>  51

Lys Ala Ser Gln Xaa Val Gly Thr Xaa Val Ala
1               5                   10


<210>  52
<211>  13
<212>  PRT
<213>  Artificial

<220>
<223>  consensus amino acid sequence of VL CDR1 of BA 11 + 67


<220>
<221>  misc_feature
<222>  (1)..(2)
<223>  Xaa can be any naturally occurring amino acid

<220>
<221>  misc_feature
<222>  (4)..(4)
<223>  Xaa can be any naturally occurring amino acid

<220>
<221>  misc_feature
<222>  (6)..(6)
<223>  Xaa can be any naturally occurring amino acid

<220>
<221>  misc_feature
<222>  (8)..(8)
<223>  Xaa can be any naturally occurring amino acid

<220>
<221>  misc_feature
<222>  (12)..(13)
<223>  Xaa can be any naturally occurring amino acid

<400>  52

Xaa Xaa Ser Xaa Ser Xaa Ile Xaa Asn Asn Tyr Xaa Xaa
1               5                   10


<210>  53
<211>  11
<212>  PRT
<213>  Artificial

<220>
<223>  consensus amino acid sequence of VL CDR1 of BA61+3+5+29+31+11

<220>
<221>  misc_feature
<222>  (1)..(1)
<223>  Xaa can be any naturally occurring amino acid

<220>
<221>  misc_feature
<222>  (5)..(11)
<223>  Xaa can be any naturally occurring amino acid

<400>  53

Xaa Ala Ser Gln Xaa Xaa Xaa Xaa Xaa Xaa Xaa
1               5                   10


<210>  54
<211>  11
<212>  PRT
<213>  Artificial

<220>
<223>  consensus amino acid sequence of VL CDR1 of BA61 + 3 + 5 + 11


<220>
<221>  misc_feature
<222>  (1)..(1)
<223>  Xaa can be any naturally occurring amino acid

<220>
<221>  misc_feature
<222>  (5)..(11)
<223>  Xaa can be any naturally occurring amino acid

<400>  54

Xaa Ala Ser Gln Xaa Xaa Xaa Xaa Xaa Xaa Xaa
1               5                   10


<210>  55
<211>  7
<212>  PRT
<213>  Artificial

<220>
<223>  VL CDR2 of BA61 + 3 + 5

<400>  55

Ser Ala Ser Tyr Arg Tyr Ser
1               5


<210>  56
<211>  7
<212>  PRT
<213>  Artificial

<220>
<223>  VL CDR2 of BA29

<400>  56

Ser Ala Ser Asn Arg Tyr Thr

1               5


<210>  57
<211>  7
<212>  PRT
<213>  Artificial

<220>
<223>  VL CDR2 of BA31

<400>  57

Trp Ala Ser Thr Arg His Thr
1               5


<210>  58
<211>  7
<212>  PRT
<213>  Artificial

<220>
<223>  VL CDR2 of BA11

<400>  58

Tyr Thr Ser Arg Leu His Ser
1               5


<210>  59
<211>  7
<212>  PRT
<213>  Artificial

<220>
<223>  VL CDR2 of BA67

<400>  59

Asp Asn Asn Lys Arg Phe Ser
1               5


<210>  60
<211>  7
<212>  PRT
<213>  Artificial

<220>
<223>  consensus amino acid sequence of VL CDR2 of all polypeptides


<220>
<221>  misc_feature
<222>  (1)..(7)
<223>  Xaa can be any naturally occurring amino acid

<400>  60

Xaa Xaa Xaa Xaa Xaa Xaa Xaa
1               5


<210>  61
<211>  7
<212>  PRT

<213>  Artificial

<220>
<223>  consensus amino acid sequence of VL CDR2 of BA61 + 3+5+29+31


<220>
<221>  misc_feature
<222>  (1)..(1)
<223>  Xaa can be any naturally occurring amino acid

<220>
<221>  misc_feature
<222>  (4)..(4)
<223>  Xaa can be any naturally occurring amino acid

<220>
<221>  misc_feature
<222>  (6)..(7)
<223>  Xaa can be any naturally occurring amino acid

<400>  61

Xaa Ala Ser Xaa Arg Xaa Xaa
1               5


<210>  62
<211>  7
<212>  PRT
<213>  Artificial

<220>
<223>  consensus amino acid sequence of VL CDR2 of BA 11 + 67


<220>
<221>  misc_feature
<222>  (1)..(6)
<223>  Xaa can be any naturally occurring amino acid

<400>  62

Xaa Xaa Xaa Xaa Xaa Xaa Ser
1               5


<210>  63
<211>  7
<212>  PRT
<213>  Artificial

<220>
<223>  consensus amino acid sequence of VL CDR2 of BA61+3+5+29+31+11


<220>
<221>  misc_feature
<222>  (1)..(2)
<223>  Xaa can be any naturally occurring amino acid

<220>
<221>  misc_feature
<222>  (4)..(7)
<223>  Xaa can be any naturally occurring amino acid

<400>  63

Xaa Xaa Ser Xaa Xaa Xaa Xaa
1               5


<210>  64
<211>  7
<212>  PRT
<213>  Artificial

<220>
<223>  consensus amino acid sequence of VL CDR2 of BA61 + 3 + 5 + 11


<220>
<221>  misc_feature
<222>  (1)..(2)
<223>  Xaa can be any naturally occurring amino acid

<220>
<221>  misc_feature
<222>  (4)..(6)
<223>  Xaa can be any naturally occurring amino acid

<400>  64

Xaa Xaa Ser Xaa Xaa Xaa Ser
1               5


<210>  65
<211>  9
<212>  PRT
<213>  Artificial

<220>
<223>  VL CDR3 of BA61 + 3 + 5

<400>  65

Gln Gln Tyr Asn Ser Tyr Pro Tyr Thr
1               5


<210>  66
<211>  9
<212>  PRT
<213>  Artificial

<220>
<223>  VL CDR3 of BA29 + 31

<400>  66

Gln Gln Tyr Ser Ser Tyr Pro Tyr Thr
1               5


<210>  67
<211>  9
<212>  PRT
<213>  Artificial

<220>
<223>  VL CDR3 of BA11

<400>  67

Gln Gln Gly Asn Thr Leu Pro Trp Thr

1               5


<210>  68
<211>  11
<212>  PRT
<213>  Artificial

<220>
<223>  VL CDR3 of BA67

<400>  68

Gly Ala Trp Asp Gly Ser Leu Arg Glu Ala Val
1               5                   10


<210>  69
<211>  11
<212>  PRT
<213>  Artificial

<220>
<223>  consensus amino acid sequence of VL CDR3 of all polypeptides


<220>
<221>  misc_feature
<222>  (1)..(4)
<223>  Xaa can be any naturally occurring amino acid

<220>
<221>  misc_feature
<222>  (6)..(10)
<223>  Xaa can be any naturally occurring amino acid

<400>  69

Xaa Xaa Xaa Xaa Gly Xaa Xaa Xaa Xaa Xaa Val
1               5                   10


<210>  70
<211>  9
<212>  PRT
<213>  Artificial

<220>
<223>  consensus amino acid sequence of VL CDR3 of BA61 + 3+5+29+31


<220>
<221>  misc_feature
<222>  (4)..(4)
<223>  Xaa can be any naturally occurring amino acid

<400>  70

Gln Gln Tyr Xaa Ser Tyr Pro Tyr Thr
1               5


<210>  71
<211>  11
<212>  PRT
<213>  Artificial

<220>

<223>  consensus amino acid sequence of VL CDR3 of BA 11 + 67


<220>
<221>  misc_feature
<222>  (1)..(4)
<223>  Xaa can be any naturally occurring amino acid

<220>
<221>  misc_feature
<222>  (6)..(6)
<223>  Xaa can be any naturally occurring amino acid

<220>
<221>  misc_feature
<222>  (8)..(10)
<223>  Xaa can be any naturally occurring amino acid

<400>  71

Xaa Xaa Xaa Xaa Gly Xaa Leu Xaa Xaa Xaa Val
1               5                   10


<210>  72
<211>  9
<212>  PRT
<213>  Artificial

<220>
<223>  consensus amino acid sequence of VL CDR3 of BA61+3+5+29+31+11


<220>
<221>  misc_feature
<222>  (3)..(6)
<223>  Xaa can be any naturally occurring amino acid

<220>
<221>  misc_feature
<222>  (8)..(8)
<223>  Xaa can be any naturally occurring amino acid

<400>  72

Gln Gln Xaa Xaa Xaa Xaa Pro Xaa Thr
1               5


<210>  73
<211>  9
<212>  PRT
<213>  Artificial

<220>
<223>  consensus amino acid sequence of VL CDR3 of BA61 + 3 + 5 + 11


<220>
<221>  misc_feature
<222>  (3)..(3)
<223>  Xaa can be any naturally occurring amino acid

<220>
<221>  misc_feature
<222>  (5)..(6)
<223>  Xaa can be any naturally occurring amino acid

<220>
<221>  misc_feature
<222>  (8)..(8)
<223>  Xaa can be any naturally occurring amino acid

<400>  73

Gln Gln Xaa Asn Xaa Xaa Pro Xaa Thr
1               5


<210>  74
<211>  15
<212>  DNA
<213>  Artificial

<220>
<223>  VH CDR1 of BA61 + 31

<400>  74
aactatgcca tgtct                                                        15


<210>  75
<211>  15
<212>  DNA
<213>  Artificial

<220>
<223>  VH CDR1 of BA29 + 3

<400>  75
aactatgcca tgtcc                                                        15


<210>  76
<211>  15
<212>  DNA
<213>  Artificial

<220>
<223>  VH CDR1 of BA11

<400>  76
aactatgtta tacac                                                        15


<210>  77
<211>  15
<212>  DNA
<213>  Artificial

<220>
<223>  VH CDR1 of BA67 + 5

<400>  77
gattatgcta tgcac                                                        15


<210>  78
<211>  15
<212>  DNA
<213>  Artificial

<220>
<223>  consensus nucleotide sequence of VH CDR1 of all polypeptides

<400>  78

raytatgyya trymy                                                        15


<210>  79
<211>  15
<212>  DNA
<213>  Artificial

<220>
<223>  consensus nucleotide sequence of VH CDR1 of BA 61 + 31 + 29 + 3

<400>  79
aactatgcca tgtcy                                                        15


<210>  80
<211>  15
<212>  DNA
<213>  Artificial

<220>
<223>  consensus nucleotide sequence of VH CDR1 of BA 11 + 67 + 5

<400>  80
raktatgyta trcac                                                        15


<210>  81
<211>  51
<212>  DNA
<213>  Artificial

<220>
<223>  VH CDR2 of BA61

<400>  81
gaaattagta ctggtggtag tcacacctac tatccagaca ctgtgacggg c               51


<210>  82
<211>  51
<212>  DNA
<213>  Artificial

<220>
<223>  VH CDR2 of BA31

<400>  82
gaaattagta ctgctggtag ttacacctac tatccagaca ctgtgacggg c               51


<210>  83
<211>  51
<212>  DNA
<213>  Artificial

<220>
<223>  VH CDR2 of BA29 + 3

<400>  83
gaaattagta ctgctggtag taacacctac tatccagaca ctgtgacggg c               51


<210>  84
<211>  51
<212>  DNA
<213>  Artificial

<220>
<223>  VH CDR2 of BA11

<400>  84
tatattaatc cttacaatga ttttactaaa tacaatgaga agttcaaagg c          51


<210>  85
<211>  51
<212>  DNA
<213>  Artificial

<220>
<223>  VH CDR2 of BA67

<400>  85
gtgattagta cttactctgg tagtacaaac tacaaccaga gctttaaggg c          51


<210>  86
<211>  51
<212>  DNA
<213>  Artificial

<220>
<223>  VH CDR2 of BA5

<400>  86
gttattagta cttactctgg tagtacaaac tacaaccaga agtttaagga c          51


<210>  87
<211>  51
<212>  DNA
<213>  Artificial

<220>
<223>  consensus nucleotide sequence of VH CDR2 of all polypeptides

<400>  87
kwdattartm ctkvydvtrr thdyachwam taymmhsasa vbktbamrgr c          51


<210>  88
<211>  51
<212>  DNA
<213>  Artificial

<220>
<223>  consensus nucleotide sequence of VH CDR2 of BA 61 + 31 + 29 + 3

<400>  88
gaaattagta ctgstggtag thacacctac tatccagaca ctgtgacggg c          51


<210>  89
<211>  51
<212>  DNA
<213>  Artificial

<220>
<223>  consensus nucleotide sequence of VH CDR2 of BA 11 + 67 + 5

<400>  89
kwkattartm cttacwmtgr twktacwaam tacaaysaga rsttyaargr c          51


<210>  90

<211>  51
<212>  DNA
<213>  Artificial

<220>
<223>  consensus nucleotide sequence of VH CDR2 of BA 67 + 5

<400>  90
gtkattagta cttactctgg tagtacaaac tacaaccaga rstttaaggr c          51


<210>  91
<211>  18
<212>  DNA
<213>  Artificial

<220>
<223>  VH CDR3 of BA61 + 31

<400>  91
actggttact ttgactac                                               18


<210>  92
<211>  18
<212>  DNA
<213>  Artificial

<220>
<223>  VH CDR3 of BA29 + 3

<400>  92
acggggtact tcgatgtc                                               18


<210>  93
<211>  24
<212>  DNA
<213>  Artificial

<220>
<223>  VH CDR3 of BA11

<400>  93
   gtcggggaaa ccccctttgc ttac                                         24


<210>  94
<211>  33
<212>  DNA
<213>  Artificial

<220>
<223>  VH CDR3 of BA5

<400>  94
acggactacc cttatttcta tgctatggac tac                              33


<210>  95
<211>  33
<212>  DNA
<213>  Artificial

<220>
<223>  VH CDR3 of BA5

<400>  95

acaaactacc cttatttcaa tggtatggac tac                                33

<210>  96
<211>  33
<212>  DNA
<213>  Artificial

<220>
<223>  consensus nucleotide sequence of VH CDR3 of all polypeptides

<220>
<221>  misc_feature
<222>  (3)..(3)
<223>  n is a, c, g, or t

<400>  96
rynrrbkamc cttatttcwm yssywtbgmy kwc                                33

<210>  97
<211>  18
<212>  DNA
<213>  Artificial

<220>
<223>  consensus nucleotide sequence of VH CDR3 of BA 61 + 31 + 29 + 3

<400>  97
ackggktact tygaykwc                                                 18

<210>  98
<211>  33
<212>  DNA
<213>  Artificial

<220>
<223>  consensus nucleotide sequence of VH CDR3 of BA 11 + 67 + 5

<400>  98
ryvrrskamc cttatttcwm yssywtkgmy tac                                33

<210>  99
<211>  33
<212>  DNA
<213>  Artificial

<220>
<223>  consensus nucleotide sequence of VH CDR3 of BA 67 + 5

<400>  99
acrractacc cttatttcwa tgstatggac tac                                33

<210>  100
<211>  33
<212>  DNA
<213>  Artificial

<220>
<223>  VL CDR1 of BA61 + 3 + 5

<400>  100
aaggccagtc agaatgtggg tactaatgta gcc                                33

<210>  101
<211>  33
<212>  DNA
<213>  Artificial

<220>
<223>  VL CDR1 of BA29

<400>  101
aaggccagtc agaatgtggg tactgctgta gcc                                    33


<210>  102
<211>  33
<212>  DNA
<213>  Artificial

<220>
<223>  VL CDR1 of BA31

<400>  102
aaggccagtc aggatgtggg tactactgta gcc                                    33


<210>  103
<211>  33
<212>  DNA
<213>  Artificial

<220>
<223>  VL CDR1 of BA11

<400>  103
agggcaagtc aggacattag caattattta aac                                    33


<210>  104
<211>  39
<212>  DNA
<213>  Artificial

<220>
<223>  VL CDR1 of BA67

<400>  104
tctggaagca gctccaacat tggaaataat tatgtctcc                              39


<210>  105
<211>  39
<212>  DNA
<213>  Artificial

<220>
<223>  consensus nucleotide sequence of VL CDR1 of all polypeptides

<400>  105
wvkgsmagym rstccrayrt krghaatamt dmtktmdmc                              39


<210>  106
<211>  33
<212>  DNA
<213>  Artificial

<220>
<223>  consensus nucleotide sequence of VL CDR1 of BA61+3+5+29+31

<400>  106
aaggccagtc agratgtggg tactrmtgta gcc                                    33


<210>  107
<211>  39
<212>  DNA
<213>  Artificial

<220>
<223>  consensus nucleotide sequence of VL CDR1 of BA11 + 67

<400>  107
wskgsaagym rstccracat trgmaataat tatktmwmc                              39


<210>  108
<211>  33
<212>  DNA
<213>  Artificial

<220>
<223>  consensus nucleotide sequence of VL CDR1 of BA61+3+5+29+31+11

<400>  108
arggcmagtc agrayrtkrg yamtdmtkta rmc                                    33


<210>  109
<211>  33
<212>  DNA
<213>  Artificial

<220>
<223>  consensus nucleotide sequence of VL CDR1 of BA61 + 3 + 5 + 11

<400>  109
arggcmagtc agrayrtkrg yamtwatkta rmc                                    33


<210>  110
<211>  21
<212>  DNA
<213>  Artificial

<220>
<223>  VL CDR2 of BA61 + 3 + 5

<400>  110
tcggcatcct accggtacag t                                                 21


<210>  111
<211>  21
<212>  DNA
<213>  Artificial

<220>
<223>  VL CDR2 of BA29

<400>  111
tcggcatcca atcggtacac t                                                 21


<210>  112
<211>  21
<212>  DNA

<213>  Artificial

<220>
<223>  VL CDR2 of BA31

<400>  112
tgggcatcca cccggcacac t                                                21


<210>  113
<211>  21
<212>  DNA
<213>  Artificial

<220>
<223>  VL CDR2 of BA11

<400>  113
tacacatcaa gattacactc a                                                21


<210>  114
<211>  21
<212>  DNA
<213>  Artificial

<220>
<223>  VL CDR2 of BA67

<400>  114
gacaataata agcgattctc a                                                21


<210>  115
<211>  21
<212>  DNA
<213>  Artificial

<220>
<223>  consensus nucleotide sequence of VL CDR2 of all polypeptides


<220>
<221>  misc_feature
<222>  (12)..(12)
<223>  n is a, c, g, or t

<400>  115
kvsrmwwmhw vnykrywcws w                                                21


<210>  116
<211>  21
<212>  DNA
<213>  Artificial

<220>
<223>  consensus nucleotide sequence of VL CDR2 of BA61+3+5+29+31

<400>  116
tsggcatccw mycggyacas t                                                21


<210>  117
<211>  21
<212>  DNA
<213>  Artificial

<220>
<223>  consensus nucleotide sequence of VL CDR2 of BA 11 + 67

<400>  117
kacamwwmwa rrykaywctc a                                                21


<210>  118
<211>  21
<212>  DNA
<213>  Artificial

<220>
<223>  consensus nucleotide sequence of VL CDR2 of BA61+3+5+29+31+11

<400>  118
   tvsrcatcmw vhykryacws w                                               21


<210>  119
<211>  21
<212>  DNA
<213>  Artificial

<220>
<223>  consensus nucleotide sequence of VL CDR2 of BA61 + 3 + 5 + 11

<400>  119
   tmsrcatcmw rmykryacws w                                               21


<210>  120
<211>  27
<212>  DNA
<213>  Artificial

<220>
<223>  VL CDR3 of BA61 + 3 + 5

<400>  120
cagcaatata acagctatcc ttacacg                                          27


<210>  121
<211>  27
<212>  DNA
<213>  Artificial

<220>
<223>  VL CDR3 of BA29 + 31

<400>  121
cagcaatata gcagctatcc gtacacg                                          27


<210>  122
<211>  27
<212>  DNA
<213>  Artificial

<220>
<223>  VL CDR3 of BA11

<400>  122
caacagggta atacgcttcc gtggacg                                          27


<210>  123

<211>  33
<212>  DNA
<213>  Artificial

<220>
<223>  nucleotide sequence of VL CDR3 of BA67

<400>  123
ggagcatggg atggcagcct gcgtgaagcg gta                                    33


<210>  124
<211>  33
<212>  DNA
<213>  Artificial

<220>
<223>  consensus nucleotide sequence of VL CDR3 of all polypeptides

<400>  124
srrsmrkrkr ryggcassyw kcskkrvrcg gta                                    33


<210>  125
<211>  27
<212>  DNA
<213>  Artificial

<220>
<223>  consensus nucleotide sequence of VL CDR3 of BA61+3+5+29+31

<400>  125
cagcaatata rcagctatcc ktacacg                                           27


<210>  126
<211>  33
<212>  DNA
<213>  Artificial

<220>
<223>  consensus nucleotide sequence of VL CDR3 of BA 11 + 67

<400>  126
srasmrkgkr atggcassct kcskkrrrcg gta                                    33


<210>  127
<211>  27
<212>  DNA
<213>  Artificial

<220>
<223>  consensus nucleotide sequence of VL CDR3 of BA61+3+5+29+31+11

<400>  127
carcarkrta ryassywtcc ktrsacg                                           27


<210>  128
<211>  27
<212>  DNA
<213>  Artificial

<220>
<223>  consensus nucleotide sequence of VL CDR3 of BA61 + 3 + 5 + 11

<400>  128

carcarkrta ayassywtcc ktrsacg                                                27

<210>  129
<211>  12
<212>  PRT
<213>  Artificial

<220>
<223>  yol-linker

<400>  129

Lys Leu Glu Glu Gly Glu Phe Ser Glu Ala Arg Val
1               5                   10


<210>  130
<211>  12
<212>  PRT
<213>  Artificial

<220>
<223>  modified yol-linker

<400>  130

Ile Leu Glu Glu Gly Glu Phe Ser Glu Ala Gly Cys
1               5                   10


<210>  131
<211>  12
<212>  PRT
<213>  Artificial

<220>
<223>  consensus amino acid sequence of the yol-linker and the modified
       yol-linker


<220>
<221>  misc_feature
<222>  (1)..(1)
<223>  Xaa can be any naturally occurring amino acid

<220>
<221>  misc_feature
<222>  (11)..(12)
<223>  Xaa can be any naturally occurring amino acid

<400>  131

Xaa Leu Glu Glu Gly Glu Phe Ser Glu Ala Xaa Xaa
1               5                   10


<210>  132
<211>  10
<212>  PRT
<213>  Artificial

<220>
<223>  GS-linker

<400>  132

Gly Gly Gly Gly Ser Gly Gly Gly Gly Ser

1               5                   10

<210>  133
<211>  35
<212>  DNA
<213>  Artificial

<220>
<223>  yol-linker

<400>  133
aagcttgaag aaggtgaatt ttcagaagca cgcgt                                35

<210>  134
<211>  795
<212>  DNA
<213>  Artificial

<220>
<223>  my BA11

<400>  134
atggcgcagg tccagttgca gcagtctgga cctgagctgg tcaagcctgg ggcttcagtg      60

aagatgtcct gcaaggcttc tggatacaca ttcactaact atgttataca ctgggtgaag     120

cagaagcctg ggcagggcct tgagtggatt ggatatatta atccttacaa tgattttact     180

aaatacaatg agaagttcaa aggcaaggcc acaccgactt cagacaaatc ctccagcaca     240

gcctacatgg agctcagcag cctgacctct gaggactctg cggtctatta ctgtacaaga     300

gtcggggaaa ccccctttgc ttactggggc caagggactc tggtcactgt ctctgcagcc     360

aaaacaacac ccccatcagt catccttgaa gaacccgggt tttcagaagc aggcctagat     420

atccagatga cacagactac atcctccctg tctgcctctc tgggagacag agtcaccatc     480

agttgcaggg caagtcagga cattagcaat tatttaaact ggtatcagca gaaaccagat     540

ggaactgtta aactcctgat ctactacaca tcaagattac actcaggagt cccatcaagg     600

ttcagtggca gtgggtctgg aacagattat tctctcacca ttagcaacct ggagcaagaa     660

gatattgcca cttacttttg ccaacagggt aatacgcttc cgtggacgtt cggtggaggc     720

accaagctgg aaatcaaacg gcctgatgct gcaccaactg tagcggccgc tggatcccat     780

caccatcacc atcac                                                      795

<210>  135
<211>  265
<212>  PRT
<213>  Artificial

<220>
<223>  my BA11

<400>  135

Met Ala Gln Val Gln Leu Gln Gln Ser Gly Pro Glu Leu Val Lys Pro
1               5                   10                  15

Gly Ala Ser Val Lys Met Ser Cys Lys Ala Ser Gly Tyr Thr Phe Thr

20                  25                  30

Asn Tyr Val Ile His Trp Val Lys Gln Lys Pro Gly Gln Gly Leu Glu
        35                  40                  45

Trp Ile Gly Tyr Ile Asn Pro Tyr Asn Asp Phe Thr Lys Tyr Asn Glu
    50                  55                  60

Lys Phe Lys Gly Lys Ala Thr Pro Thr Ser Asp Lys Ser Ser Ser Thr
65                  70                  75                  80

Ala Tyr Met Glu Leu Ser Ser Leu Thr Ser Glu Asp Ser Ala Val Tyr
                85                  90                  95

Tyr Cys Thr Arg Val Gly Glu Thr Pro Phe Ala Tyr Trp Gly Gln Gly
            100                 105                 110

Thr Leu Val Thr Val Ser Ala Ala Lys Thr Thr Pro Pro Ser Val Ile
        115                 120                 125

Leu Glu Glu Pro Gly Phe Ser Glu Ala Gly Leu Asp Ile Gln Met Thr
    130                 135                 140

Gln Thr Thr Ser Ser Leu Ser Ala Ser Leu Gly Asp Arg Val Thr Ile
145                 150                 155                 160

Ser Cys Arg Ala Ser Gln Asp Ile Ser Asn Tyr Leu Asn Trp Tyr Gln
                165                 170                 175

Gln Lys Pro Asp Gly Thr Val Lys Leu Leu Ile Tyr Tyr Thr Ser Arg
            180                 185                 190

Leu His Ser Gly Val Pro Ser Arg Phe Ser Gly Ser Gly Ser Gly Thr
        195                 200                 205

Asp Tyr Ser Leu Thr Ile Ser Asn Leu Glu Gln Glu Asp Ile Ala Thr
    210                 215                 220

Tyr Phe Cys Gln Gln Gly Asn Thr Leu Pro Trp Thr Phe Gly Gly Gly
225                 230                 235                 240

Thr Lys Leu Glu Ile Lys Arg Pro Asp Ala Ala Pro Thr Val Ala Ala
                245                 250                 255

Ala Gly Ser His His His His His His
            260                 265

<210>  136
<211>  804
<212>  DNA
<213>  Artificial

<220>
<223>  gs BA11

<400>  136
atggcgcagg tccagttgca gcagtctgga cctgagctgg tcaagcctgg ggcttcagtg      60

aagatgtcct gcaaggcttc tggatacaca ttcactaact atgttataca ctgggtgaag     120

cagaagcctg ggcagggcct tgagtggatt ggatatatta atccttacaa tgattttact     180

aaatacaatg agaagttcaa aggcaaggcc acactgactt cagacaaatc ctccagcaca     240

gcctacatgg agctcagcag cctgacctct gaggactctg cggtctatta ctgtacaaga     300

gtcggggaaa ccccctttgc ttactggggc caagggactc tggtcactgt ctctgcagcc     360

aaaacaacac ccccatcagt caagcttggt ggcggcggat caggaggagg cggttctgca     420

cgcgtagata tccagatgac acagactaca tcctccctgt ctgcctctct gggagacaga     480

gtcaccatca gttgcagggc aagtcaggac attagcaatt atttaaactg gtatcagcag     540

aaaccagatg gaactgttaa actcctgatc tactacacat caagattaca ctcaggagtc     600

ccatcaaggt tcagtggcag tgggtctgga acagattatt ctctcaccat tagcaacctg     660

gagcaagaag atattgccac ttacttttgc caacagggta atacgcttcc gtggacgttc     720

ggtggaggca ccaagctgga aatcaaacgg cctgatgctg caccaactgt agcggccgct     780

ggatcccatc accatcacca tcac                                            804

<210>  137
<211>  268
<212>  PRT
<213>  Artificial

<220>
<223>  gs BA11

<400>  137

Met Ala Gln Val Gln Leu Gln Gln Ser Gly Pro Glu Leu Val Lys Pro
1               5                   10                  15

Gly Ala Ser Val Lys Met Ser Cys Lys Ala Ser Gly Tyr Thr Phe Thr
            20                  25                  30

Asn Tyr Val Ile His Trp Val Lys Gln Lys Pro Gly Gln Gly Leu Glu
        35                  40                  45

Trp Ile Gly Tyr Ile Asn Pro Tyr Asn Asp Phe Thr Lys Tyr Asn Glu
    50                  55                  60

Lys Phe Lys Gly Lys Ala Thr Leu Thr Ser Asp Lys Ser Ser Ser Thr
65                  70                  75                  80

Ala Tyr Met Glu Leu Ser Ser Leu Thr Ser Glu Asp Ser Ala Val Tyr
                85                  90                  95

Tyr Cys Thr Arg Val Gly Glu Thr Pro Phe Ala Tyr Trp Gly Gln Gly

100                 105                 110

Thr Leu Val Thr Val Ser Ala Ala Lys Thr Thr Pro Pro Ser Val Lys
        115                 120                 125

Leu Gly Gly Gly Gly Ser Gly Gly Gly Gly Ser Ala Arg Val Asp Ile
    130                 135                 140

Gln Met Thr Gln Thr Thr Ser Ser Leu Ser Ala Ser Leu Gly Asp Arg
145                 150                 155                 160

Val Thr Ile Ser Cys Arg Ala Ser Gln Asp Ile Ser Asn Tyr Leu Asn
                165                 170                 175

Trp Tyr Gln Gln Lys Pro Asp Gly Thr Val Lys Leu Leu Ile Tyr Tyr
            180                 185                 190

Thr Ser Arg Leu His Ser Gly Val Pro Ser Arg Phe Ser Gly Ser Gly
        195                 200                 205

Ser Gly Thr Asp Tyr Ser Leu Thr Ile Ser Asn Leu Glu Gln Glu Asp
    210                 215                 220

Ile Ala Thr Tyr Phe Cys Gln Gln Gly Asn Thr Leu Pro Trp Thr Phe
225                 230                 235                 240

Gly Gly Gly Thr Lys Leu Glu Ile Lys Arg Pro Asp Ala Ala Pro Thr
                245                 250                 255

Val Ala Ala Ala Gly Ser His His His His His His
            260                 265

<210>  138
<211>  541
<212>  PRT
<213>  Artificial

<220>
<223>  bv BA11

<400>  138

Met Ala Gln Val Gln Leu Gln Gln Ser Gly Pro Glu Leu Val Lys Pro
1               5                   10                  15

Gly Ala Ser Val Lys Met Ser Cys Lys Ala Ser Gly Tyr Thr Phe Thr
            20                  25                  30

Asn Tyr Val Ile His Trp Val Lys Gln Lys Pro Gly Gln Gly Leu Glu
        35                  40                  45

Trp Ile Gly Tyr Ile Asn Pro Tyr Asn Asp Phe Thr Lys Tyr Asn Glu
    50                  55                  60

Lys Phe Lys Gly Lys Ala Thr Leu Thr Ser Asp Lys Ser Ser Ser Thr
65                  70                  75                  80

Ala Tyr Met Glu Leu Ser Ser Leu Thr Ser Glu Asp Ser Ala Val Tyr
                85                  90                  95

Tyr Cys Thr Arg Val Gly Glu Thr Pro Phe Ala Tyr Trp Gly Gln Gly
            100                 105                 110

Thr Leu Val Thr Val Ser Ala Ala Lys Thr Thr Pro Pro Ser Val Lys
        115                 120                 125

Leu Glu Glu Gly Glu Phe Ser Glu Ala Arg Val Asp Ile Gln Met Thr
    130                 135                 140

Gln Thr Thr Ser Ser Leu Ser Ala Ser Leu Gly Asp Arg Val Thr Ile
145                 150                 155                 160

Ser Cys Arg Ala Ser Gln Asp Ile Ser Asn Tyr Leu Asn Trp Tyr Gln
                165                 170                 175

Gln Lys Pro Asp Gly Thr Val Lys Leu Leu Ile Tyr Tyr Thr Ser Arg
            180                 185                 190

Leu His Ser Gly Val Pro Ser Arg Phe Ser Gly Ser Gly Ser Gly Thr
        195                 200                 205

Asp Tyr Ser Leu Thr Ile Ser Asn Leu Glu Gln Glu Asp Ile Ala Thr
    210                 215                 220

Tyr Phe Cys Gln Gln Gly Asn Thr Leu Pro Trp Thr Phe Gly Gly Gly
225                 230                 235                 240

Thr Lys Leu Glu Ile Lys Arg Pro Asp Ala Ala Pro Thr Val Gly Gly
                245                 250                 255

Gly Gly Ser Gly Gly Gly Gly Ser Glu Phe Gly Gly Gly Gly Ser Gly
            260                 265                 270

Gly Gly Gly Ser Met Ala Gln Val Gln Leu Gln Gln Ser Gly Pro Glu
        275                 280                 285

Leu Val Lys Pro Gly Ala Ser Val Lys Met Ser Cys Lys Ala Ser Gly
    290                 295                 300

Tyr Thr Phe Thr Asn Tyr Val Ile His Trp Val Lys Gln Lys Pro Gly
305                 310                 315                 320

Gln Gly Leu Glu Trp Ile Gly Tyr Ile Asn Pro Tyr Asn Asp Phe Thr
                325                 330                 335

Lys Tyr Asn Glu Lys Phe Lys Gly Lys Ala Thr Leu Thr Ser Asp Lys
            340                 345                 350

Ser Ser Ser Thr Ala Tyr Met Glu Leu Ser Ser Leu Thr Ser Glu Asp
        355                 360                 365

Ser Ala Val Tyr Tyr Cys Thr Arg Val Gly Glu Thr Pro Phe Ala Tyr
    370                 375                 380

Trp Gly Gln Gly Thr Leu Val Thr Val Ser Ala Ala Lys Thr Thr Pro
385                 390                 395                 400

Pro Ser Val Lys Leu Glu Glu Gly Glu Phe Ser Glu Ala Arg Val Asp
                405                 410                 415

Ile Gln Met Thr Gln Thr Thr Ser Ser Leu Ser Ala Ser Leu Gly Asp
            420                 425                 430

Arg Val Thr Ile Ser Cys Arg Ala Ser Gln Asp Ile Ser Asn Tyr Leu
        435                 440                 445

Asn Trp Tyr Gln Gln Lys Pro Asp Gly Thr Val Lys Leu Leu Ile Tyr
    450                 455                 460

Tyr Thr Ser Arg Leu His Ser Gly Val Pro Ser Arg Phe Ser Gly Ser
465                 470                 475                 480

Gly Ser Gly Thr Asp Tyr Ser Leu Thr Ile Ser Asn Leu Glu Gln Glu
                485                 490                 495

Asp Ile Ala Thr Tyr Phe Cys Gln Gln Gly Asn Thr Leu Pro Trp Thr
            500                 505                 510

Phe Gly Gly Gly Thr Lys Leu Glu Ile Lys Arg Pro Asp Ala Ala Pro
        515                 520                 525

Thr Val Ala Ala Ala Gly Ser His His His His His His
    530                 535                 540

<210>  139
<211>  6
<212>  PRT
<213>  Artificial

<220>
<223>  consensus amino acid sequence of VH CDR3 of all polypeptides

<220>
<221>  misc_feature
<222>  (1)..(6)
<223>  Xaa can be any naturally occurring amino acid

<400>  139

Xaa Xaa Xaa Xaa Xaa Xaa
1               5


<210>  140
<211>  8
<212>  PRT
<213>  Artificial

<220>
<223>  consensus amino acid sequence of VH CDR3 of all polypeptides


<220>
<221>  misc_feature
<222>  (1)..(8)
<223>  Xaa can be any naturally occurring amino acid

<400>  140

Xaa Xaa Xaa Xaa Xaa Xaa Xaa Xaa
1               5


<210>  141
<211>  8
<212>  PRT
<213>  Artificial

<220>
<223>  consensus amino acid sequence of VH CDR3 of BA 11 + 67 + 5


<220>
<221>  misc_feature
<222>  (1)..(7)
<223>  Xaa can be any naturally occurring amino acid

<400>  141

Xaa Xaa Xaa Xaa Xaa Xaa Xaa Tyr
1               5


<210>  142
<211>  6
<212>  PRT
<213>  Artificial

<220>
<223>  consensus amino acid sequence of VH CDR3 of BA61 + 31+29+3+11


<220>
<221>  misc_feature
<222>  (1)..(1)
<223>  Xaa can be any naturally occurring amino acid

<220>
<221>  misc_feature
<222>  (3)..(3)
<223>  Xaa can be any naturally occurring amino acid

<220>
<221>  misc_feature
<222>  (5)..(6)
<223>  Xaa can be any naturally occurring amino acid

<400>  142

Xaa Gly Xaa Phe Xaa Xaa
1               5


<210>  143
<211>  11
<212>  PRT
<213>  Artificial

<220>
<223>  consensus amino acid sequence of VL CDR1 of all polypeptides


<220>
<221>  misc_feature
<222>  (1)..(2)
<223>  Xaa can be any naturally occurring amino acid

<220>
<221>  misc_feature
<222>  (4)..(11)
<223>  Xaa can be any naturally occurring amino acid

<400>  143

Xaa Xaa Ser Xaa Xaa Xaa Xaa Xaa Xaa Xaa Xaa
1               5                   10


<210>  144
<211>  11
<212>  PRT
<213>  Artificial

<220>
<223>  consensus amino acid sequence of VL CDR1 of BA 11 + 67


<220>
<221>  misc_feature
<222>  (1)..(2)
<223>  Xaa can be any naturally occurring amino acid

<220>
<221>  misc_feature
<222>  (4)..(5)
<223>  Xaa can be any naturally occurring amino acid

<220>
<221>  misc_feature
<222>  (7)..(7)
<223>  Xaa can be any naturally occurring amino acid

<220>
<221>  misc_feature
<222>  (10)..(11)
<223>  Xaa can be any naturally occurring amino acid

<400>  144

Xaa Xaa Ser Xaa Xaa Ile Xaa Asn Tyr Xaa Xaa
1               5                   10


<210>  145

<211>  9
<212>  PRT
<213>  Artificial

<220>
<223>  consensus amino acid sequence of VL CDR3 of all polypeptides


<220>
<221>  misc_feature
<222>  (1)..(9)
<223>  Xaa can be any naturally occurring amino acid

<400>  145

Xaa Xaa Xaa Xaa Xaa Xaa Xaa Xaa Xaa
1               5


<210>  146
<211>  9
<212>  PRT
<213>  Artificial

<220>
<223>  consensus amino acid sequence of VL CDR3 of BA 11 + 67


<220>
<221>  misc_feature
<222>  (1)..(5)
<223>  Xaa can be any naturally occurring amino acid

<220>
<221>  misc_feature
<222>  (7)..(9)
<223>  Xaa can be any naturally occurring amino acid

<400>  146

Xaa Xaa Xaa Xaa Xaa Leu Xaa Xaa Xaa
1               5